# EUROPEAN PATENT SPECIFICATION

(11) **EP 2 644 615 B1**
(45) Date of publication and mention of the grant of the patent: **05.04.2017**
(21) Application number: 13162214.4
(22) Date of filing: 08.02.2007
(51) Int. Cl.: C07H 21/04, G01N 33/53, C12Q 1/68, G01N 33/543, G01N 33/58

(54) **SERS nanotag assays**
SERS-Nanoetikett-Assays
Bioessais utilisant des nanomarqueurs SERS

(30) Priority: 08.02.2006 US 771766 P; 24.07.2006 US 832917 P; 20.12.2006 US 870963 P
(43) Date of publication of application: 02.10.2013
(62) Divisional of application: 07763259.4
(73) Proprietor: Becton Dickinson and Company, Franklin Lakes, NJ 07417-1880 (US)
(72) Inventor: Xu, Hongxia, Castro Valley, CA 94552 (US); Doering, William E., Moutain View, CA 94043 (US); Cromer, Remy, San Jose, CA 95130 (US); Natan, Michael, Los Altos, CA 94024 (US); Penn, Sharron Gaynor, San Carlos, CA 94070 (US); Sha, Michael, San Jose, CA 95130 (US)
(74) Representative: Eder, Michael

(56) References cited:
- WO-A2-03/035829
- WO-A2-2004/007767
- US-A1- 2004 043 512
- US-A1- 2006 046 311
- CHOWDHURY M H ET AL: "The use of surface enhanced Raman spectroscopy (SERS) in a competitive affinity binding assay for the detection of prions", PROCEEDINGS OF THE 25TH. ANNUAL INTERNATIONAL CONFERENCE OF THE IEEE ENGINEERING IN MEDICINE AND BIOLOGY SOCIETY. CANCUN, MEXICO, SEPT. 17 - 21, 2003; [ANNUAL INTERNATIONAL CONFERENCE OF THE IEEE ENGINEERING IN MEDICINE AND BIOLOGY SOCIETY. EMBS], NE, vol. 4, 17 September 2003 (2003-09-17), pages 3049-3051, XP010693906, DOI: 10.1109/IEMBS.2003.1280784 ISBN: 978-0-7803-7789-9
- HARRI HÄRMÄ: "Particle technologies in diagnostics", TECHNOLOGY REVIEW, vol. 126, 1 April 2002 (2002-04-01), XP055223581, ISBN: 978-952-4570-78-7

## Description

### TECHNICAL FIELD

The present invention is directed toward a method and system for the use of Surface Enhanced Raman Scattering nanotags (SERS nanotags) to create a variety of assay platforms.

### BACKGROUND OF THE INVENTION

Particles, and magnetic particles in particular, are extensively used in diagnostic assays as solid phase capture or detection species. Microparticle-based assays can be divided into two main categories: homogeneous (separation-free) and heterogeneous assays.

In a homogeneous (separation-free) assay format, binding reactants are mixed and measured without any subsequent washing step prior to detection. The advantages of such a system are fast solution-phase kinetics, a simple assay format, simpler instrumentation as well as lower costs because of fewer assay steps, low volumes and low waste. Homogeneous immunoassays do not require physical separation of bound and free analyte and thus may be faster and easier to perform then heterogeneous immunoassays. Homogeneous immunoassay systems using small sample size, low reagent volume and short incubation times, provide fast turnaround time. Disadvantages of this type of assay can be limited dynamic range and sensitivity. Since there is no separation of free analyte before signal detection, sensitivity might be further compromised. Also, interferences could cause a high background signal by interaction between the sample and capture or detection reagents. Homogeneous assays are the preferred assay format in high throughput screening platforms such as AlphaScreen, SPA, fluorescent polarization and flow cytometry based assays, as well as in diagnostic assays such as particle agglutination assays with nephelometry or turbidimetry as the detection methods.

Heterogeneous immunoassays requiring the separation of free analyte and of unbound detector and in certain instances may be more versatile than homogeneous assays. The wash or physical separation steps eliminate most interfering substances and in general do not interfere with the detection/quantification step. Stepwise heterogeneous assays are possible which allow for larger sample size, which in turn improves sensitivity and yields wider dynamic range than the standard assay curves. The disadvantages of heterogeneous immunoassays are that they are much more labor-intensive, time-consuming and typically require dedicated analyzers. In addition, automated heterogeneous systems require more complicated designs or multiple instruments to accommodate wash and separation steps. Many clinical analyzers use magnetic microparticles for heterogeneous diagnostic assays to selectively bind and then separate the analyte of interest from its surrounding matrix using a magnetic field (The Immunoassay Handbook (2001) Nature Publ, London). Analyzers based on this format include the ACS 180 and Immuno I from Bayer Diagnostics, the Access from Beckman Coulter and the Elecsys from Roche Diagnostics.

Traditionally, only one biomarker has been used for diagnosis of a disease with homogeneous assays such as described above: for example Prostate Specific Antigen (PSA) is the marker of choice for prostate cancer even though it may have poor specificity (Lu-Yao et al. (2003) J Natl Cancer Inst. 95:1792-1797). With the advent of proteomics, there is a belief that diagnostic assays involving several biomarkers will increase the accuracy, specificity and aid in discerning between different diseases that exhibit similar symptoms. Screening for several markers, as is already done in the genomics field, provides better diagnostic or prognostic information than screening for one biomarker alone. See for example, Genomic Health's Oncotype Dx Breast Cancer Assay (2005) (www.genomichealth.com).

Assays designed to shorten the time from sampling to diagnosis are equally important in emergency room or point-of-care settings. For example, Biosite Inc. offers a lateral flow immunoassay for the rapid diagnosis and assessment of the severity of heart failure and risk stratification in acute coronary syndromes (Biosite Website (2005) http://www.biosite.com/products/cardiac.aspx.) Simpler, more accurate, and faster assay technologies are therefore needed to clinically implement the many biomarkers newly identified through proteomics research. The ability to quantify three or more proteins simultaneously in a fast, no-wash, no-sample preparation bioassay would dramatically improve the state of the art in disease diagnosis and prognosis, as well as in disease progression monitoring.

The present invention is directed toward overcoming one or more of the problems discussed above.

### SUMMARY OF THE INVENTION

The present invention provides several methods and systems for the use of Surface Enhanced Raman Scattering nanotags (SERS nanotags) to create homogeneous (no-wash), heterogeneous or sequence detection assay platforms. Multiplexed assay platforms are also disclosed. In general, the assay is useful for clinical proteomics. Assay platforms suitable for use within a biological matrix, for example within whole blood or serum are also disclosed. The assay formats described herein may be used to detect any analyte of interest including but not limited to the detection of cells, viruses, bacteria, proteins, DNA, RNA, or small molecules in any type of biological (animal or plant kingdom) or environmental samples including but not limited to whole blood or serum, occult samples, urine, feces, air, drinking water, phage, any organism, multicellular clumps of cells, for example, cancer tissue homogenate.

In a first aspect, the present invention provides a method of detecting an analyte of interest which comprises:
providing capture particles derivatized with said analyte;
providing SERS nanotag detection particles conjugated with a first molecule capable of selectively binding said analyte;
contacting a sample which may contain the analyte of interest with the capture and SERS nanotag detection particles, thereby forming both an analyte/SERS nanotag detection particle complex and a capture particle/SERS nanotag detection particle complex;
concentrating the capture particle/SERS nanotag detection particle complex; and
detecting the Raman spectrum of a Raman reporter molecule associated with the SERS nanotag detection particles.

In certain embodiments of the method as claimed herein, the capture particles are magnetic particles. In some of these embodiments, the concentration step of said method is performed on a magnetic particle concentrator.

In a second aspect, the present invention provides an assay system for detecting an analyte of interest according to a method as defined herein, comprising capture particles derivatized with said analyte and SERS nanotag detection particles conjugated with a first molecule capable of selectively binding said analyte. Optionally, the capture particles are magnetic particles.

The Detailed Description below includes the following subsections:
A. A general discussion of SERS nanotags for biological assays.
B. Homogenous or heterogeneous sandwich immunoassays featuring capture antibodies associated with the inside wall of a test vial.
C. Homogenous or heterogeneous sandwich immunoassays featuring magnetic capture particles.
D. SERS / magnetic bead hybridization to support a SERS magnetic DNA assay.
E. Competitive SERS/Magnetic bead assay.
F. Nucleotide Sequence Detection assays.

The use of magnetic capture particles in certain of the assays discussed below will enable easy integration with existing clinical analyzers. The use of SERS nanotags (as will be elaborated on below) will enable the use of a no-wash, multiplexed, assay format. It is important to note that there is an industry wide emerging trend toward miniaturization, typically by use of microfluidic and nanofluidic technologies, which will impact the diagnostics field by allowing the reduction of reagent and sample volume as well as reducing the reaction time involved in each experiment. Miniaturization may thus potentially lower the total cost-per-experiment However, to achieve the benefits of miniaturization, new sets of challenges such as the need for precise and accurate liquid handling procedures must be overcome. The assay method and platform discussed herein in many instances requires no liquid handling, thus circumventing the micro and nano-fluidics issues. The assay methods and platforms of the present invention will handle and probe any volume size equally well.

### BRIEF DESCRIPTION OF THE DRAWINGS

Fig. 1 is a schematic diagram of a SERS nanotag suitable for implementation of select embodiments of the present invention;
Fig. 2 is a graph comparison of the SERS spectra of SERS nanotags having five different reporter molecules;
Fig. 3 is a schematic illustration of a sequential assay consistent with the present disclosure;
Fig. 4 is a graphic representation of an IL-4 dose response curve;
Fig. 5 is a schematic and photographic illustration of a homogeneous assay consistent with the present disclosure;
Fig. 6 is a graphic representation of an IL-4 dose response in the presence of excess nanotags;
Fig. 7 is a graphic representation of the spectra of surface bound BPE tags;
Fig. 8 is a graphic representation of homogeneous assay IL-4 dose response;
Fig. 9A-C are various graphic representations of Raman spectra obtained in the presence of biological fluids;
Fig. 10 is a graphic representation of Raman spectrum recovery in biological fluids;
Fig. 11 is a schematic illustration of an assay consistent with the present disclosure;
Fig. 12 is a graphic representation of the dose response for a "no wash" IL-4 assay;
Fig. 13 is a graphic representation of the assay of Fig. 12 with reduced incubation time and limited detection particles;
Fig. 14 is a graphic representation of the assay of Fig. 12 with reduced incubation time and limited detection particles;
Fig. 15 is a graphic representation of an optimized assay IL-4 dose response curve;
Fig. 16 is a graphic representation of spectra recovery from biological matrices;
Fig. 17 is a graphic representation of detection tag and buffer screening results;
Fig. 18 is a graphic representation of detection tag and buffer screening results;
Fig. 19 is a graphic representation of a CRP dose response curve with DPY detection tags;
Fig. 20 is a graphic representation of the results of a multiplexed assay;
Fig. 21 is a dose response curve for CRP;
Fig. 22 is a schematic diagram of SERS Magnetic Bead Hybridization assay;
Fig. 23 is a graphic representation of the detection of DNA consistent with the present disclosure; and
Fig. 24 is a graphic representation of the results of a SERS DNA Magnetic Assay Titration.
Figs. 25 is a schematic illustration of a competitive assay format.
Fig. 26 is a schematic diagram of a nucleotide sequence detection assay.
Fig. 27 is a representative probe suitable for use in a nucleotide sequence detection assay.
Fig. 28 is a graphic representation of the results of a nucleotide sequence detection assay.
Fig. 29 is a representation of representative probes suitable for use in a nucleotide sequence detection assay.
Fig. 30 is a graphic representation of an assay titration using the probes of Fig. 29.
Fig. 31 is a graphic representation of assay results for select PCR amplicons.
Fig. 32 is a graphic representation of assay results after serial dilutions.
Fig. 33 is a schematic diagram of a nucleotide sequence detection assay.
Fig. 34 is a graphic representation of the results of the assay of Fig. 33.
Fig. 35 is a schematic illustration of an assay featuring nucleotide cutting.
Fig. 36 is a schematic illustration of an assay featuring RCA.
Fig. 37 is a graphic representation of the results of the assay of Fig. 36.

### DETAILED DESCRIPTION OF THE INVENTION

### A. General discussion of SERS Nanotags for Biological Assays.

A homogeneous multiplexed assay platform is typically designed around a two-particle system. A capture particle is conjugated with an antibody to capture the antigen of interest from the biological sample. The detection particle may be a SERS-nanotag particle as described below also conjugated with a detection antibody with binding affinity for the antigen of interest. The SERS nanotag includes a Raman reporter molecule as described below. In the presence of the antigen of interest both the capture particle and the detection particle are bound to form a SERS active, 2-particle, immunocomplex. SERS nanotags offer at least three intrinsic advantages as detection tags. (1) They can be excited in the near-IR, and thus are compatible with whole blood measurement. (2) SERS nanotags resist photobleaching which allows for higher laser powers and longer data acquisition times, resulting in more sensitive measurements. (3) A large number of distinct tags exist, enabling highly multiplexed assays.

SERS nanotags are novel, nanoparticulate optical detection tags based on surface enhanced Raman scattering (SERS) (Mulvaney et al. (2003) Langmuir 19:4784-4790; Natan, US Patent No. 6,514,767). Raman scattering (Long (2002) The Raman Effect; A Unified Treatment of the Theory of Raman Scattering by Molecules. John Wiley & Sons Ltd, Chichester; Modern Techniques in Raman Spectroscopy (1996) John Wiley & Sons Ltd, Chichester; Analytical Applications of Raman Spectroscopy (1999) Blackwell Science Ltd, Malden, MA.) SERS is a laser-based optical spectroscopy that, for molecules, generates a fingerprint-like vibrational spectrum with features that are much narrower than typical fluorescence. Raman scattering can be excited using monochromatic far-red or near-IR light, photon energies which are too low to excite the inherent background fluorescence in biological samples. Since Raman spectra typically cover vibrational energies from 300-3500 cm⁻¹, it could be possible to measure a dozen (or more) tags simultaneously, all with a single light source. However, normal Raman spectra are very weak, limiting utility for bioanalytical chemistry. In SERS, molecules in very close proximity to nanoscale roughness features on noble metal surfaces (gold, silver copper) give rise to million- to trillion-fold increases [known as enhancement factor (EF)] in scattering efficiency (Moskovits (1985) Rev. Mod. Phys. 57:783-826; Otto et al. (1992) J. Phys. Cond. Mat. 4:1143-1212; Campion and Kambhampati (1998) Chem. Soc. Rev. 27:241-249;Tian et al. (2002) J. Phys. Chem. B 106:9463-9483; CAS Online Search, April 2004), More importantly, SERS can also be used to detect molecules adsorbed to individual metal nanoparticles (Emory et al. (1998) J. Am. Chem. Soc. 120:8009-8010; Moyer et al. (2000) J. Am. Chem. Soc. 122:5409-5410), and has been used to demonstrate detection of single molecules (Nie and Emory (1997) Science 275:1102-1106; Kneipp et al. (1997) Phys. Rev. Lett. 78:1667-1670; Michaels et al. (1999) J. Am. Chem. Soc. 121:9932-9939; Xu et al. (1999) Phys. Rev. Lett. 83:4357-4360; Goulet et al. (2003) Anal. Chem. 75:1918-1923).

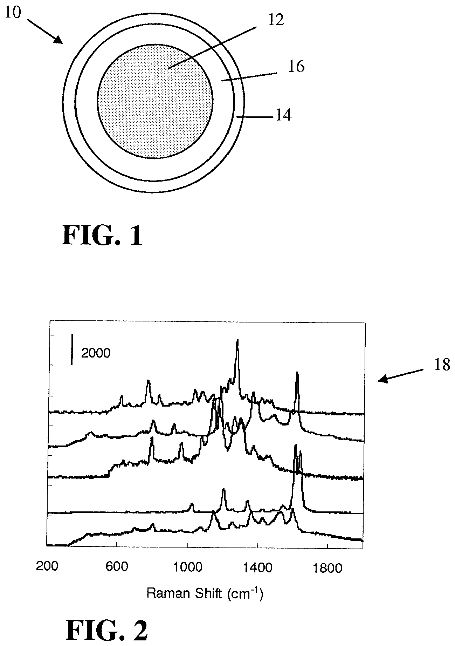

A typical SERS nanotag 10 is shown in Fig. 1. The illustrated SERS nanotag 10 includes a metal nanoparticle core 12, and a SiO₂ (glass) shell 14. Other materials including but not limited to various types of polymers may also be used as an encapsulant or shell consistent with the present invention. Details concerning the use, manufacture and characteristics of a typical SERS nanotag are included in U.S. Patent No. 6,514,767, entitled "Surface Enhanced Spectroscopy-Active Composite Nanoparticles." Although the invention is described in terms of SERS nanotags prepared from single nanoparticle cores 12, it is to be understood that nanoparticle core clusters or aggregates may be used in the preparation of SERS nanotags. Methods for the preparation of clusters of aggregates of metal colloids are known to those skilled in the art. The use of sandwich-type particles as described in U.S. Patent No. 6,861,263, is also contemplated.

The nanoparticle core 12 may be of any material known in the art to be Raman-enhancing. The nanoparticles cores 12 may be isotropic or anisotropic. Anisotropic nanoparticles may have a length and a width. In some embodiments, the length of an anisotropic nanoparticle is the dimension parallel to the aperture in which the nanoparticle was produced. In the case of anisotropic nanoparticles, in some embodiments, the nanoparticle has a diameter (width) of 350 nm or less. In other embodiments, the nanoparticle has a diameter of 250 nm or less and in some embodiments, a diameter of 100 nm or less. In some embodiments, the width is between 15 nm to 300 nm. In some embodiments, the nanoparticle has a length of about 10-350 nm.

Nanoparticles suitable to be the core of a SERS nanotag include colloidal metal, hollow or filled nanobars, magnetic, paramagnetic, conductive or insulating nanoparticles, synthetic particles, hydrogels (colloids or bars), and the like. The nanoparticles used in the present invention can exist as single nanoparticles, or as clusters or aggregates of the nanoparticles.

It will be appreciated by one of ordinary skill in the art that nanoparticles can exist in a variety of shapes, including but not limited to spheroids, rods, disks, pyramids, cubes, cylinders, nanohelixes, nanosprings, nanorings, rod-shaped nanoparticles, arrow-shaped nanoparticles, teardrop-shaped nanoparticles, tetrapod-shaped nanoparticles, prism-shaped nanoparticles, and a plurality of other geometric and non-geometric shapes. Another class of nanoparticle that has been described include those with internal surface area. These include hollow particles and porous or semi-porous particles. Moreover, it is understood that methods to prepare particles of these shapes, and in certain cases to prepare SERS-active particles of these shapes, have been described in the literature. While it is recognized that particle shape and aspect ratio can affect the physical, optical, and electronic characteristics of nanoparticles, the specific shape, aspect ratio, or presence/absence of internal surface area does not bear on the qualification of a particle as a nanoparticle.

A nanoparticle also includes a nanoparticle in which the metal includes an additional component, such as in a core-shell particle. For example, Ag core/Au shell particles, like those described in J. Am. Chem. Soc. 2001, 123, 7961, or Au core/Ag shell particles, or any core-shell combination involving SERS-active metals, can be used. Other combinations suitable for use in core-shell particles are included in this invention, such as Au- or Ag-nanoparticle functionalized silica/alumina colloids, Au- or Ag- functionalized TiO₂ colloids, Au nanoparticle capped-Au nanoparticles (see, for example, Mucic, et al., J. Am. Chem. Soc. 1998, 120, 12674), Au nanoparticle-capped TiO₂ colloids, particles having and Si core with a metal shell ("nanoshells"), such as silver-capped SiO₂ colloids or gold-capped SiO₂ colloids. (See, e.g. Jackson, et al., 2004 Proc Natl Acad Sci USA. 101(52):17930-5; Talley, et al., Nano Letters (2005)). Hollow nanoparticles such as hollow nanospheres and hollow nanocrystals may also be utilized in the SERS nanotags.

Each SERS nanotag is encoded with a unique reporter 16, comprising an organic or inorganic molecule at the interface between the nanoparticle core and shell of glass or other suitable encapsulant. This approach to detection tags leverages the strengths of Raman scattering as a high-resolution molecular spectroscopy tool and the enhancements associated with SERS, while bypassing the shortcomings often encountered when making stand alone SERS substrates such as difficult reproducibility and lack of selectivity. SERS nanotags exhibit intense spectra (enhancement factors in excess of 10⁶) with the 633 and 785 nm excitation wavelengths that are excellent for avoiding intrinsic background fluorescence in biological samples such as whole blood and in matrices like glass and plastic. The glass coating, which is essentially SERS-inactive, stabilizes the particles against aggregation, prevents the reporter from diffusing away, prevents competitive adsorption of unwanted species, and provides an exceptionally well-established surface to which biomolecules can be conjugated for bioassay development (Aslam and Dent (1998) Bioconjugation: Protein Coupling Techniques for the Biomedical Sciences. Grove's Dictionaries Inc, New York, NY).

Multiple unique flavors of tags are available. Fig. 2 shows a graph 18 of the spectra of five unique SERS tags, with clearly differentiated features, which can be utilized for a multiplexed assay. In a multiplexed assay, a single spectrum will be acquired, and it will be necessary to quantitatively separate that spectrum into its components. For spectra deconvolution and quantification of the individual components an enhanced commercial software package may be used to carry out a linear least squares analysis using pure spectra from each tag as a standard.

### B. Sandwich Immunoassays with Capture Antibodies on an Inside Wall of a Test Vial.

Certain heterogeneous and homogeneous immunoassays described herein feature derivatizing an inside wall of the test vial, with capture antibody. A whole blood or serum assay may be feasible with this format particularly if excess "unbound" tags are removed before Raman spectrum acquisition. It has been demonstrated under this format that SERS tags can be read in presence of neat plasma or whole blood and that excess tag removal is not required if the detection system provides for a suitably narrow depth of field.

Representative test vial wall based assays have been prepared according to the method detailed in Example 1 below. Other methods are equally suitable for preparing or performing an assay consistent with the present disclosure.

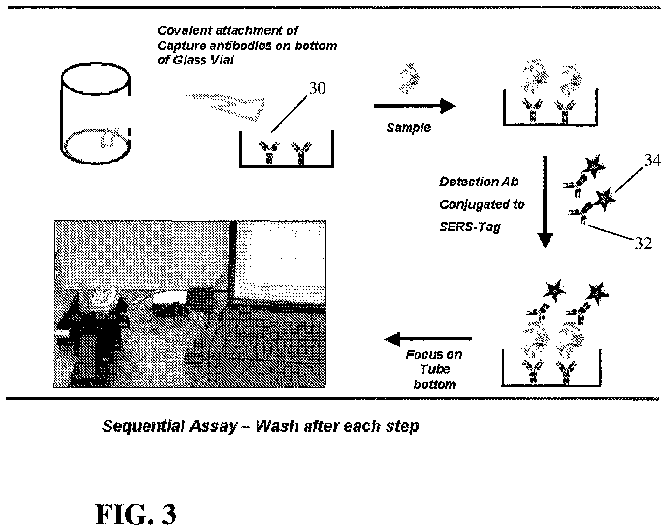

A representative sequential (heterogeneous) test vial wall assay is schematically illustrated in Fig. 3. The capture antibody 30 is covalently attached to the bottom of a container and the detection antibody 32 is covalently attached to a SERS nanotag 34.

In the sequential assay, sample was added to the vial and incubated for 2.5 hours. Unbound analyte is washed off before incubation with the SERS nanotags for another two hours. Raman spectra, focusing on the bottom wall of the glass vial, were taken before removal of unbound SERS nanotags and again after washing away excess tags.

This assay format was used to screen antibody and select antibody pairs. After a set of mix-and-match experiments, a monoclonal anti-IL-2 antibody was chosen as the capture antibody and conjugated to derivatized vial. A polyclonal was chosen as the detection antibody.

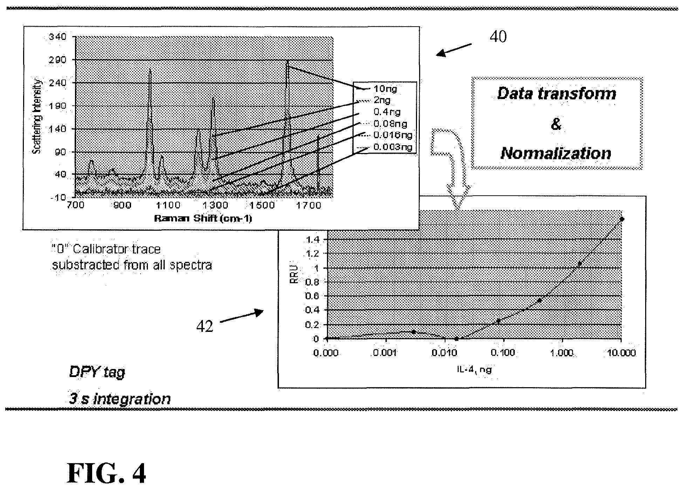

Graphs 40 and 42 of Fig. 4 illustrate the standard curve obtained in the sequential format assay described above. Data taken over 20 readings of the blank sample (washing after each step, no analyte) gave a limit of detection of about 100pg/ml.

Raman spectra were collected for each IL-4 dose. The Raman spectra shown in Fig. 4 were mathematically reduced to a number called Relative Raman Unit (RRU), using a classical least-squares-method. This number, proportional to peak intensity is normalized to a stored reference spectrum of the same SERS nanotag.

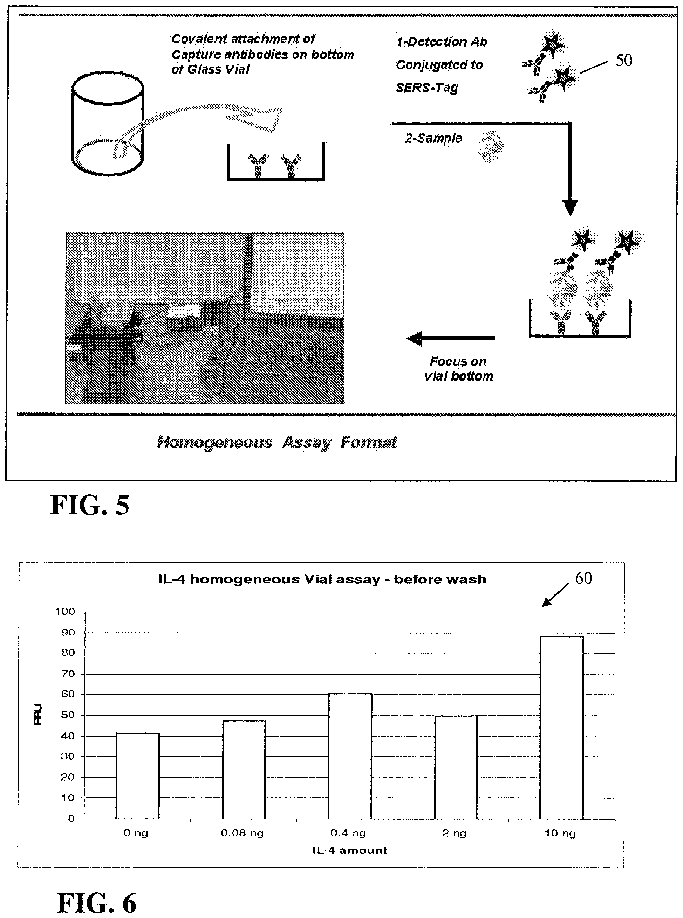

An alternative homogeneous assay format is schematically illustrated in Fig. 5. Detection conjugate 50 was loaded into the vial prior addition of the sample (analyte). After one hour incubation, the vial was read 1) in presence of unbound tags, 2) after removal the supernatant (unbound tags) and again 3) after washing the vials (removal of non-specifically bound tags).

Chart 60 of Fig. 6 illustrates the dose response curve of the Fig. 5 homogeneous assay before removal of excess unbound tags. Compared to the sequential-wash assay, a higher signal for all IL-4 concentrations was observed along with a depressed dynamic range indicating a contribution of the unbound tags in solution. This "free tag" interference is possibly attributed to the large depth of field of the Raman reader.

To demonstrate that high background is due to free tags in solution, a polylysine coated glass cover slip was loaded with a sub-monolayer of BPE tags. A suspension of a different tag (DPY) was added to the cover slip. Focusing on the surface the cover slip with a 50x objective (collection depth of ∼2 microns; Renishaw Raman Microscope), Raman spectra were collected at different time intervals.

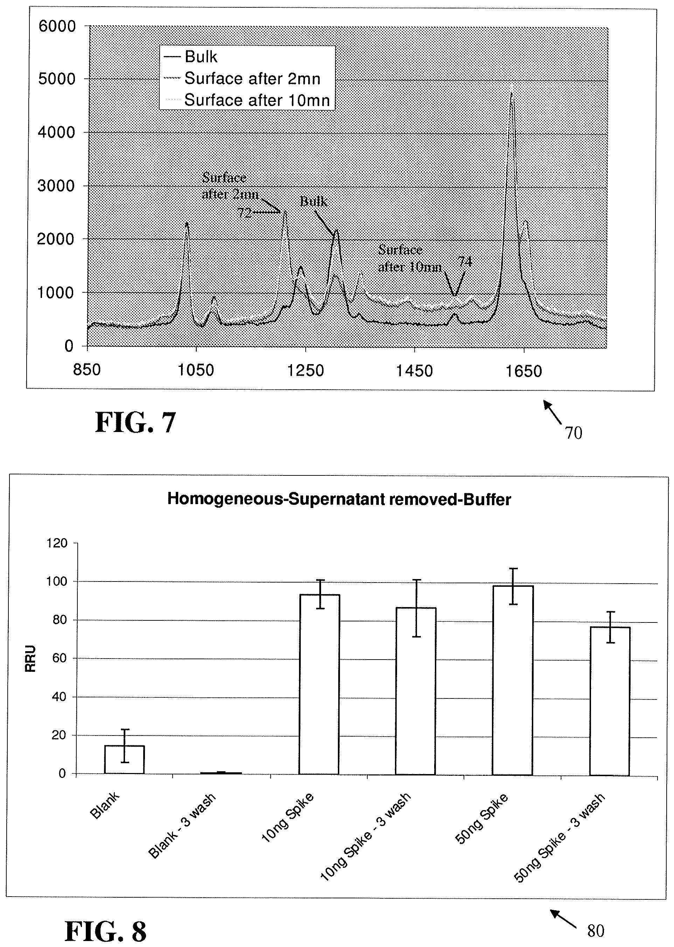

Graph 70 of Fig. 7 illustrates the Raman spectra of a cover slip surface prepared as described above after 2 minutes (trace 72) and 10 minutes (trace 74) as well as the spectrum of the suspension of DPY tags above the glass surface (Bulk spectrum 76 after 11 minutes). The "2mn-surface spectrum" is essentially a BPE spectrum with very little contribution from DPY tags. As expected, the DPY spectral contribution is increasing over time as tags start to bind to the surface via ionic interactions. Spectra taken from the solution above the surface reveal only presence of DPY tags. This demonstrates that surface-bound tags can be discriminated from tags in solution when an objective with a narrow depth-of-field is used. The Raman reader collection depth used in the collection of the data of Fig. 4, (currently a 1000-fold deeper than the 50x) does not allow discrimination between surface and solution tags, thus requiring removal of the supernatant (and wash) before taking Raman Spectra.

When excess tags are washed off, background signal is dramatically reduced as shown in Graph 80 of Fig. 8. Also, the overall signal of the "washed vials" was observed to vary widely, indicating that the "wash buffer" is not optimized. No effort in wash buffer optimization was undertaken.

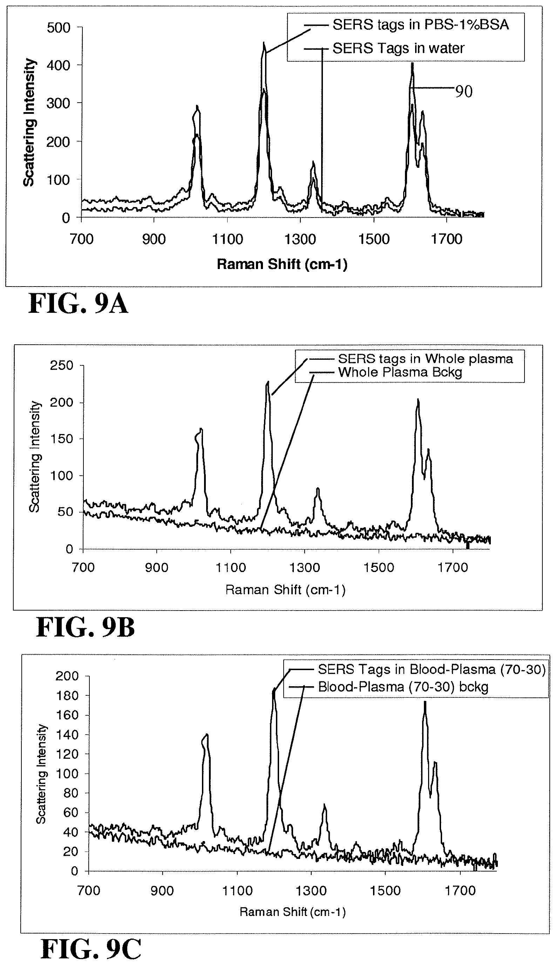

Before investigating the effect of a biological matrix on assay performance, it was demonstrated that the SERS nanotags can be read in the presence of plasma or blood. Figs. 9A, 9B and 9C describe the effect of water and 1% BSA in PBS (Fig. 9A), whole plasma (Fig. 9B) and a plasma and whole blood mixture (Fig. 9C) on spectral imaging of SERS nanotags bound to the bottom wall of polylysine coated glass vials. It should first be noted that both neat plasma and whole blood have no significant Raman scattering intensity between wavenumbers 700 and 1800 other than discrete reduction of scattering intensity with increasing wavenumber. This could be attributed to energy absorption by the sample resulting in reduction of energy for nanotag excitation. Alternatively, the biological matrix could wash-off some SERS nanotags from the vial surface.

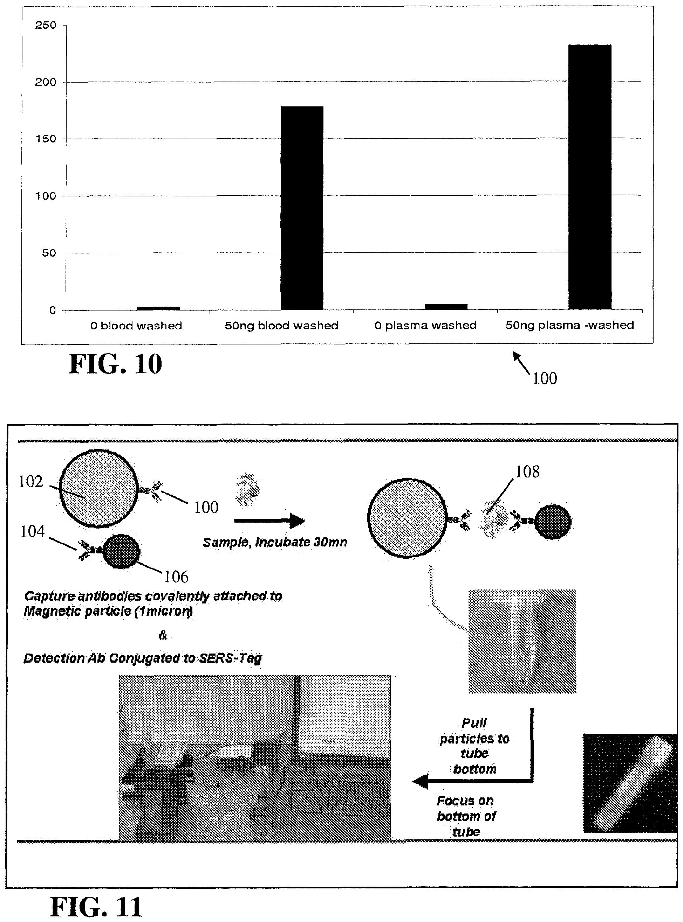

The effect of human plasma and blood on assay performance has also been investigated. Plasma or blood concentrations at 30% of total assay volume negatively affected assay performance by giving high background signal and poor or no spike recovery - before and after wash. Graph 100 of Fig. 10 however, shows the ability of the assay to recover a IL-4 spike (50ng) in 16% whole blood or 16% plasma.

### Example 1:

Representative method for the preparation of an assay featuring the capture of detection tags on an inside test vial wall.

### Materials & Reagents

- Readings were taken on a commercially available Raman Spectrum detection device.
- Dynabeads M-270 Carboxylic Acid, 2.8um, Pdt#143.05, Dynal Biotech (Oslo, Norway).
- BcMag Silica-Modified Magnetic Beads, 1um, Cat. Number FF-101 were from BioClone Inc. (San Diego, CA).
- Anti-human IL-4 Monoclonal (MAB 604) and Goat Polyclonal (AF 204Na) were obtained from R&D Systems (Minneapolis, MN).
- Anti-human CRP antibodies, Clones 265-10032 and 265-10036 were obtained from OEM Concepts (Toms River, NJ).
- Detection antibody was covalently attached to SERS Nanotag using standard protocol developed at Nanoplex REF.
- APTMS, EDC and Succinic Anhydride were obtained from Sigma.
- (3-Glycidoxypropyl)- Trimethoxysilane. United Chemical Technologies.
- Bovine Serum Albumin was from Pierce.
- Glass Vials, 12x32mm Part # CTV-1002 are from ChromTech, Inc. (MN).

### Buffers

- MES 50 mM, pH 4.5
- PBS
- Magnetic Particle Storage buffer: 0.1% BSA, PBS
- Assay buffer for Vial based assays: 1% BSA, 0.5% PEG, 0.05% Tween 20, PBS
- Assay buffer for magnetic bead based assays: 5% BSA, 0.5% PEG, 0.05% Tween 20, PBS
- Wash buffers: 1% BSA, 0.25% Tween 20, PBS

### Methods and Protocols

### General procedure for antibody conjugation to Dynabeads

1. Wash 1mg Dynabeads M-270 Carboxylic Acid with 200µl MES (twice).
2. Re-suspend with 100µl MES into Eppendorf tube (500µl).
3. Add 30µl Antibody (1mg/ml).
4. Add 100µl MES to the tube.
5. Mix well and incubate with slow tilt rotation at room temperature for 30 minutes.
6. Immediately before use dissolve EDC in cold 100 mM MES, pH 5 to a concentration of 100mg/ml.
7. Add 10 µl EDC solution (1 mg) to the Dynabeads/ligand suspension. Mix well.
8. Incubate for 2 hours at RT with slow tilt rotation.
9. Block with 200µl Ethanolamine 50mM, PBS (3mg/ml).
10. Incubate for 0.5 hour at RT with slow tilt rotation.
11. Wash 1x with 300µl 1%BSA, PBS.
12. Block overnight with 1%BSA, PBS at 4C.
13. Wash 2x 0.1BSA, 0.05 Tween in PBS.
14. Store conjugate at 1mg/ml in 1%BSA, PBS.

### GLASS VIAL DERIVATIZATION: APTMS

1. Add 80µl solution of 2% APTMS/2% water in EtOH to vial. Just enough volume to cover bottom surface-max volume = 80µl.
2. React 30 minutes at room temperature.
3. Remove solution.
4. Add EtOH, 500µl and set on hot plate for 30 minutes (lowest setting).
5. Wash and rinse once with EtOH.
6. Wash 3 times with DI water.
7. Store in 200µl water.

### SUCCINIC ANHYDRIDE DERIVATIZATION OF APTMS DERIVATIZED VIALS

1. Exchange water of APTMS coated particle for Borate buffer.
2. Prepare solution of succinic anhydride (40mg/ml) in DMSO.
3. Add 100µl of 40µl of succinic anhydride solution in 1ml Borate. Incubate at RT for 30 minutes.
4. Repeat step 3.
5. Wash 3 times with DI water.
6. Store in DI water at 1mg/ml.

### ANTIBODY CONJUGATION TO THIOLATED-SERS TAGS

1. Prepare a sulfo-SMCC solution at 1 mg/ml in water.
2. Add 100 µl of sulfo-SMCC per 50 µg of antibody.
3. Vortex mixture and allow to react for 30-45 minutes.
4. Purify maleimide-activated antibody solution using a Sephadex G25 microspin desalting column (Amersham) according to manufacturer directions.
5. Add 100 µl of PBS to the thiolated tags (4x1011 particles in 1ml water) to give a 1 mM PBS solution.
6. Add purified maleimide-activated protein to the solution. Mix well and react for 2 hours at room temperature. Particles can be gently agitated or occasionally vortexed to facilitate the reaction.
7. Prepare a 10 mg/ml solution of MESA in water. Add 50 µL to the tags and react for 20-30 minutes.
8. Add 25 µL of a 5% BSA solution to the tags and centrifuge to purify. Spin at -1500 RCF (3800 RPM on Jouan 14 microcentrifuge) for 12 minutes.
9. Resuspend particles in 1 ml of 0.1x PBS + 25 µL of 5% BSA. Centrifuge again. Repeat one or two more times (for a total of 3 or 4 centrifugation steps).
10. Resuspend particles to desired concentration in desired buffer containing 0.1% BSA.

### ANTIBODY CONJUGATION TO APTMS AND CARBOXY-APTMS COATED VIALS

1. Take 10µl of Antibody stock and dilute into 1ml PBS - Conjugation to vial will be done at 10ug/ml.
2. Add 100µl into vials.
3. Incubate for 30 minutes.
4. Prepare EDC solution (1M, 200mg/ml DMSO).
5. Add 10µl of EDC solution into vials containing Abs.
6. Store at 4C overnight.
7. Add 100µl (PBS, BSA 1%) - Blocking.
8. Orbit-Shake for 1 hour.
9. Wash with 3x200µl PBS, 0.1% BSA.

### SEQUENTIAL IL-4 ASSAY PROCEDURE -VIAL ASSAY

1. Dilute IL 4 with 0.1% BSA/ PBS solution - IL-4 stock solution at 100ng/ml.
2. Add 100µl to relevant vials.
3. Incubate on plate-shaker for 2.5 hours @RT.
4. Aspirate solutions.
5. Wash 3x200µl PBS, 0.05 %BSA, 0.25% Tween.
6. Dilute 10x IL4-SERS conjugate to appropriate concentrations.
7. Add 100µl into relevant vials.
8. Incubate for 2 hours on plate shaker.
9. Aspirate and read on Ocean Optics Reader.

### HOMOGENEOUS IL-4 ASSAY PROCEDURE - VIAL

1. Dilute 10x IL4-SERS conjugate to appropriate concentration using 0.1% BSA/PBS.
2. Add 100µl into relevant vials.
3. Add 100 µl sample - reaction buffer, plasma or blood.
4. Dilute IL-4 in PBS, 0.1%BSA.
5. Add 100µl to relevant vials.
6. Incubate on plate-shaker for 1 hour @RT.
7. Remove solution.
8. Read spectra on Ocean Optics reader.
9. Optional: Wash 3x200µl PBS, 0.05 %BSA, 0.25% Tween.
10. Read spectra on Ocean Optics reader.

### C. Sandwich Immunoassays Employing Magnetic Capture Particles.

Certain heterogeneous and homogeneous immunoassays described herein feature the use of magnetic capture particles. The combined properties of magnetic capture particles and SERS nanotag detection particles results in a bioanalytical platform which avoids the use of robotics, extensive or complicated sample preparation and analytical separations, while permitting simple, quantitative, fast (real time) and multiplexed protein measurements. Fig. 11 schematically illustrates one assay format described herein. A capture antibody 100 is covalently attached to a magnetic particle 102 and the detection antibody 104 is covalently attached to a SERS nanotag 106. Both capture and detection particles are loaded into a vessel, for example a polypropylene tube (pre-diluted with the appropriate dilution buffer) followed by the sample. An antigen associated with a chemical, protein or substance of interest 108 causes the formation of a 2-particle immunocomplex by binding with the capture and detection particles. After incubation, for example on an end-over-end mixing wheel, the tube is placed on a magnetic particle concentrator to pull down the 2-particle immunocomplex plus free magnetic particles to the bottom of the tube. The tube is then placed on the SERS reader and the laser is focused on the magnetic particles before recording the Raman spectrum from reporter molecules associated with the SERS nanotags. Accordingly, magnetic particles allow not to separate and wash in discrete steps, but to concentrate the captured analytes and complexed detection particles at a specific location within the reaction vessel while still in presence of the sample matrix and excess free detection particles. An assay implemented with magnetic particles and SERS nanotags may be particularly useful for detection of proteins in whole blood or serum. Examples demonstrating variations of the preparation and use of this bioanalytical platform assay are discussed in the following sections.

Representative magnetic capture particle based assays have been prepared according to the method detailed in Example 2 below. Other methods are equally suitable for preparing or performing an assay consistent with this aspect of the present disclosure.

According to the method of Example 2, both capture and detection particles are loaded into a polypropylene tube (pre-diluted with the appropriate dilution buffer) followed by the sample. After incubation on an end-over-end mixing wheel, the tube is placed on a magnetic particle concentrator to pull down the magnetic particle to the bottom of the tube. The tube is then placed on the SERS reader and the laser is focused on the magnetic particles before recording the Raman spectrum.

A no-wash (homogeneous) sandwich immunoassay has been prepared for C-Reactive Protein using a magnetic particle derivatized with the capture antibody and a detection antibody conjugated to SERS nanotags as reporter as described above. Results indicate that the 2-particle complex can be analyzed in presence of biological matrices, without wash and using low cost detection systems.

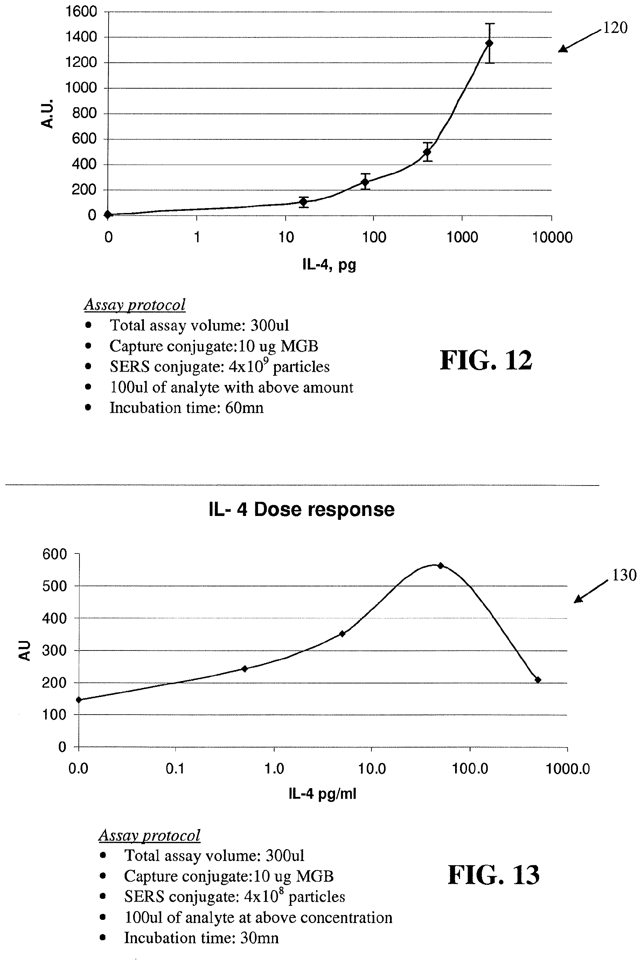

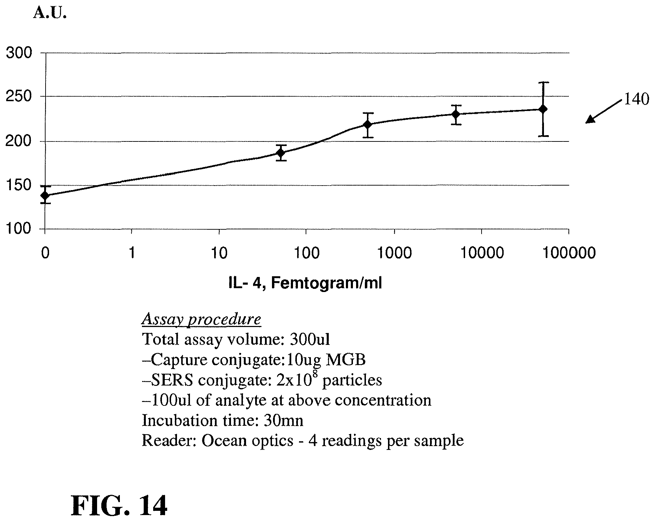

The results of an IL-4 magnetic glass coated bead-based assay are illustrated by the dose-response curve 120 of Fig. 12. These IL-4 preliminary results were obtained with high concentration of particles and with a relatively long incubation time of 1 hour. Preferably, the sensitivity of the IL-4 assay can be optimized resulting in a reduction of the incubation time to 30 minutes or substantially less. As shown in Graph 130 of Fig. 13, a reduction of incubation time to 30minutes and a 10-fold reduction of detection particles did not negatively affect assay performance other than the signal amplitude and an expected change in the dynamic range of the test. A further 2 fold-reduction of concentration in detection particles results in a femtogram per ml assay sensitivity range as shown in Graph 140 of Fig. 14. In the Fig. 14 example, non-specific signal is decreasing with decreasing amount of particles but accordingly overall signal intensity is reduced as well. Nevertheless, these results show that assay sensitivity in the vicinity of 100 femtogram/ml are achievable.

The above described one step, 30 minute, no-wash assay compares very favorably with the sensitivity (∼100fg/ml) of the commercially available assays using the same antibodies but requiring multiple steps taking place over an entire day including multiple wash steps and 200µl sample volume.

Numerous factors can impact assay sensitivity, including but not limited to:
- Biocompatible coating on capture and detection particle surface,
- Antibody amount conjugated to particle,
- Assay buffer composition,
- Capture and detection particle concentration, and
- Reader sensitivity
Certain of these factors, and the optimization of these factors are discussed in detail below.

### Biocompatible coating:

Reduction of non-specific particle - particle interaction can be achieved by judicious choice of biocompatible coatings which are usually selected from natural or synthetic polymers of different lengths, charges and polarity.

### Antibody concentration on SERS nanotags:

A set of antibody SERS nanotag conjugates were prepared using 10 and 100-fold less antibody during the conjugation process. The resulting conjugates were tested and compared to non-reduced concentration conjugates. Both conjugates did not show activity most probably because of the overall reduction of detection antibody concentration in the system. However the minimum amount of antibody suitable for preparation of an effective assay has not yet been determined.

### Assay buffer composition:

Buffer composition and buffer additives can have a dramatic effect on assay performance. Several qualities of assay blockers have been investigated. A preferred assay buffer for the CRP assay contains 5% BSA, 0.05% PEG, 0.05% Tween 20 in PBS. For an IL-4 assay a 1% BSA solution in PBS is suitable.

### Capture and Detection particle concentration:

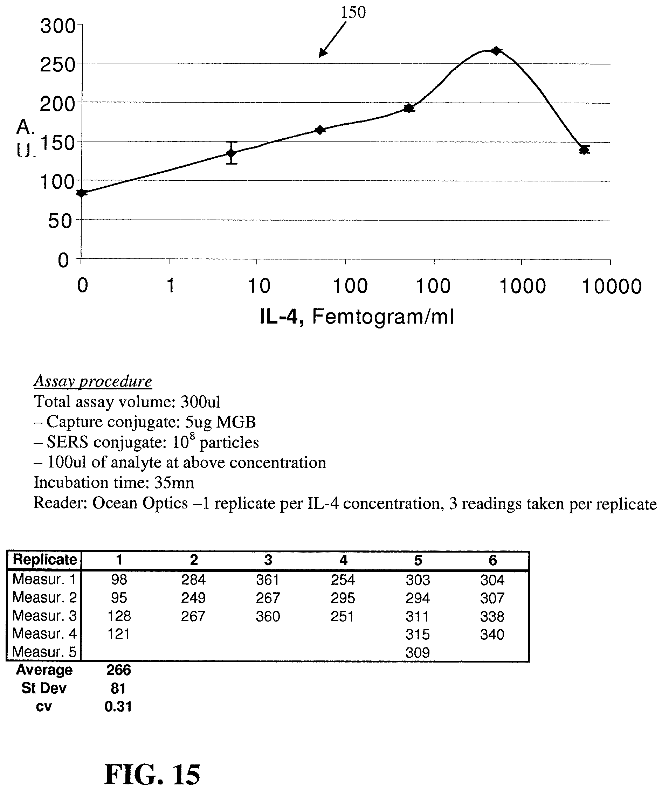

Multidimensional matrix experiments have been performed investigating primarily the effect of capture and detection particle concentration on assay performance. Graph 150 of Fig. 15 illustrates a best dose response curve obtained during this optimization process. Note that the reagent concentration has been further reduced compared to the experiment described in Fig. 14 and the dynamic range of the assay has accordingly been further reduced. The result of the Fig. 15 assay is good for a "no-wash assay" compared to commercially available products and shows that sensitivity of 10 femtogram/ml is achievable

Assay sensitivity may be defined as the Limit of Detection calculated from the Mean value plus 2 standard deviations of 18 replicates of a zero calibrator (blank). Alternatively, 6 "zero calibrators" may be analyzed in parallel with proper control of timing. The following Table 1 gives the non-specific signal (obtained from multiple measurements of 6 replicates of a "blank" or "zero calibrator". A coefficient of variance (cv) of ∼31% was determined, which is consistent with the average non-specific signal variability seen on a regular basis across independent experiments run.

**Table 1: Relative intensity (in Arbitrary Units) of 6 replicates of a "blank" sample (minimum of 3 measurements for each sample).**

| **Replicate** | **1** | **2** | **3** | **4** | **5** | **6** |
|---|---|---|---|---|---|---|
| Measur. 1 | 98 | 284 | 361 | 254 | 303 | 304 |
| Measur. 2 | 95 | 249 | 267 | 295 | 294 | 307 |
| Measur. 3 | 128 | 267 | 360 | 251 | 311 | 338 |
| Measur. 4 | 121 | | | | 315 | 340 |
| Measur. 5 | | | | | 309 | |
| **Average** | **266** | | | | | |
| **St Dev** | **81** | | | | | |
| **cv** | **0.31** | | | | | |

This relatively high coefficient of variance shown in Table 1 indicates that more sufficient control of the non-specific interaction between the 2 particles is desirable, although reduction of their amounts appears to minimize the overall signal due to non-specific binding.

Assuming that this cv can be applied across all assays run, a Limit of Detection below 500 femtogram/ml may be determined for most of the dose-response curves. Here, the Limit of Detection was estimated from the Mean value plus 3 standard deviations of the zero calibrator (blank) since 6 replicates instead of 18 replicates was used to make the determination.

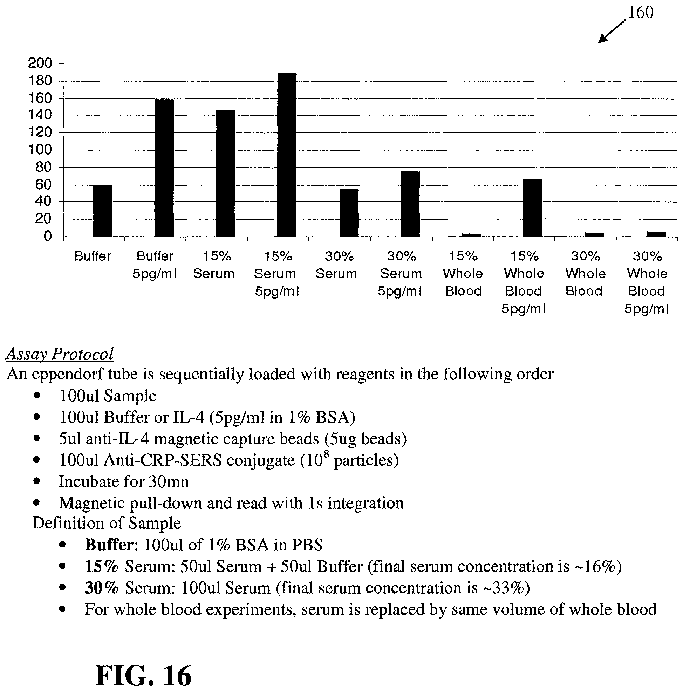

As described in detail below, an IL-4 assay may be carried out in presence of a biological matrix (for example a mid-range IL-4 spike may be recovered in 15% and 30% whole blood and serum) without further optimization. As illustrated in Graph 160 of Fig. 16, SERS tags forming immune complexes with magnetic capture particles may be detected both in serum and whole blood. The "15% serum - no spike" result gave approximately the same signal as 500fg of IL- 4, yielding a concentration of approximately 10pg/ml of IL-4 in the serum sample (∼500fg in 50µl serum). The recovery of spiked IL-4 at (5pg/ml) is very low and increasing sample concentration drastically reduced both the specific and the non-specific signal. The best recovery (∼32%) was obtained in the "15% serum" sample.

Assays prepared with magnetic capture particles and SERS nanotag detection particles are particularly well suited for the performance of multiplexed assays. In order to run a multiplexed dose response curve for combined IL-4 and CRP, unique detection tags for each analyte must be prepared. In addition, the 2 assays need to function in the same buffer (harmonized assay conditions). An example assay was prepared with a first BPE SERS nanotag. DPY was selected as a second tag and detection antibodies for both IL-4 and CRP were conjugated to both tags. Furthermore, harmonized buffer conditions need to be identified since the preferred assay buffer for CRP is PBS containing 5% BSA, 0.05% PEG and 0.05% Tween 20 whereas for IL-4 the preferred buffer is 1% BSA in PBS.

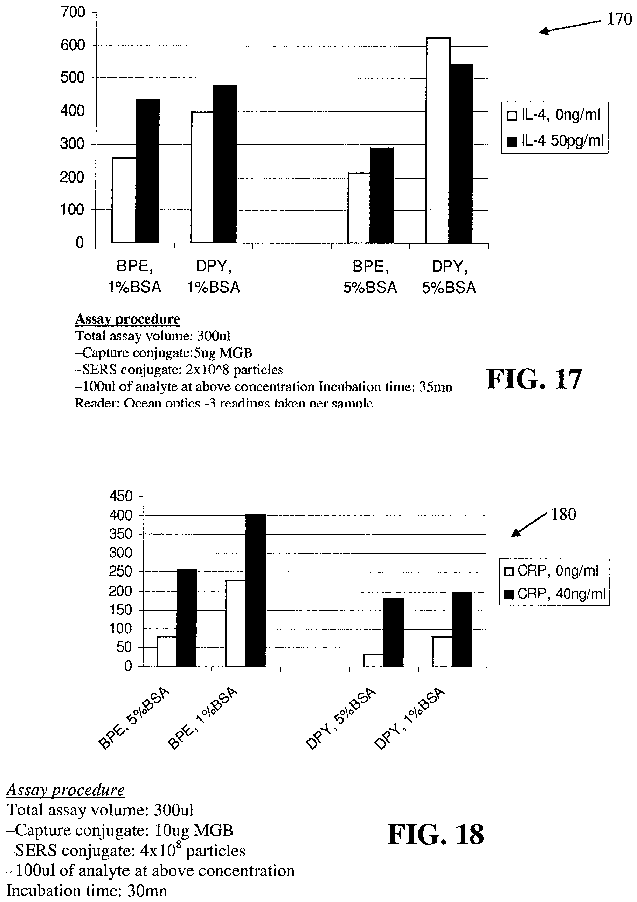

Graph 170 of Fig. 17 summarizes the detection tag and buffer screening results for the IL-4 portion of a multiplexed assay and confirms that BPE and 1% BSA are the conditions of choice for this assay. The difference in signal intensity between 50pg/ml and absence of IL-4 is the largest for the BPE tag in 1% BSA. The DPY tag conjugate does not give a sufficient positive signal in 5% BSA (PBS containing 5% BSA, 0.05% PEG and 0.05% Tween 20).

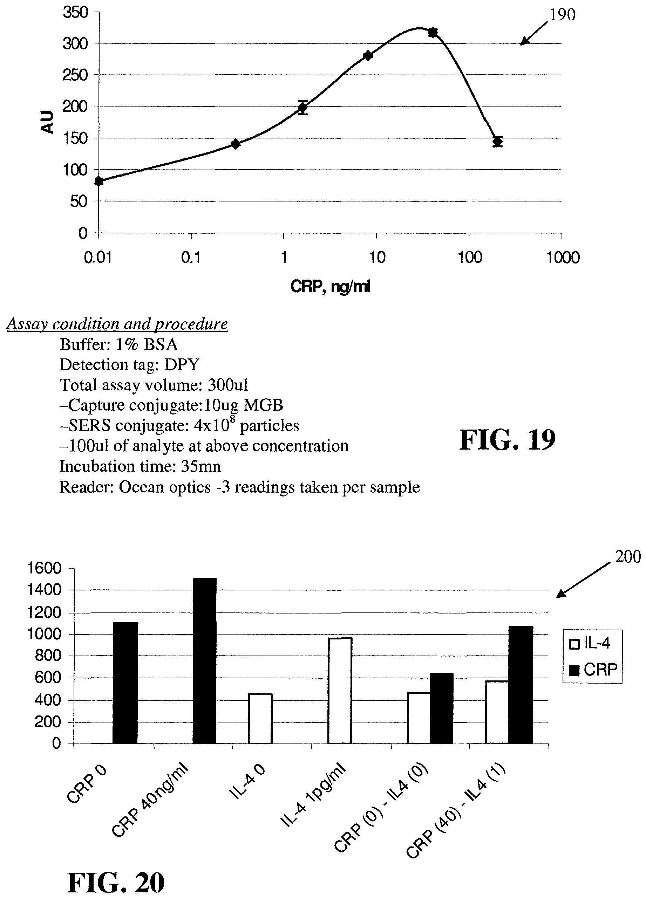

Graph 180 of Fig. 18 summarizes the detection tag and buffer screening results for the CRP portion of a multiplexed assay, both detection tag and both assay conditions work reasonably well, with the best conditions being the BPE tag in 5% BSA. A suitable multiplexed assay may be based on the BPE tag for IL-4 and 1%BSA as the preferred choice of buffer. Accordingly, the DPY tag may be selected for CRP. As shown in Graph 190 of Fig. 19, the CRP portion of the assay remains functional under these less than ideal conditions. Graph 200 of Fig. 20 shows signal intensities of negative and positive controls (monoplex) for both CRP and IL-4 followed by the multiplexed experiment (double negative and double positive). A reduction of signal intensity in 3 out of 4 assay signal was noticed in the multiplexed assay. Only the IL-4 (0) signal intensity remained similar to that demonstrated in the monoplex assay. Nevertheless the recovery of a positive reading remained the same for the CRP assay (Difference of ∼400units in both mono- and multiplexed assay) while it was strongly suppressed in the IL-4 case. This indicates inter-assay interference. For example, the presence of excess of CRP based complexes might prevent accurate reading of much smaller number of IL-4 specific complexes. Further investigations are necessary to understand and resolve the reason underlying the overall signal suppression and the low recovery of the IL-4 spike.

### Example 2:

The following example of assays prepared with magnetic capture particles are provided for illustrative purposes only. Applicant has performed experiments on an assay such as depicted in Fig. 11.

### 1. Antibody conjugation to thiolated-SERS nanotags

A solution of sulfo-SMCC (100 µL, 1 mg/ml in water) was added to solution of antibody (50 µg, 50 µl) in PBS. After 30 minutes, the maleimide-activated antibody was purified using a Sephadex G25 microspin desalting column (Amersham). The purified conjugate was then added to thiolated-SERS tags (4x10¹⁰ particles in 100 µL PBS). After 2 hours at room temperature, the reaction was quenched for 30mn with a solution of MESA (50 µL, 10 mg/ml) followed by a 5% BSA solution (25 µL). The tag conjugates were washed, purified by centrifugation and resuspended in 0.1% BSA at the desired concentration in desired buffer.

### 2. Carboxylation of Magnetic Glass coated beads

Magnetic glass coated beads from Bioclone (4mg) were washed 2 times with ethanol (1ml) before treatment with a solution of 2% APTMS (3-aminopropyltrimethoxysilane) in ethanol (1ml) for 30 mn at room temperature. The beads were washed via magnetic pulldown and treated with ethanol (500 µl) on a hot plate (50C) for 30mn. After washing with ethanol and water (3 times), the magnetic particles were stored in water at 4mg/ml. Carboxylation was done in borate buffer by treatment with a succinic anhydride solution in DMSO (20 µl, 40mg/ml) in DMSO for 30 mn. The carboxylated beads were washed and stored in water.

### 3. General procedure or antibody conjugation to Carboxylated Magnetic Glass coated beads and Dynabeads

Carboxylated magnetic glass coated beads or Dynabeads M-270 carboxylic acid (1mg) in PBS (100 µl) were treated with antibody (30µl, 1mg/ml) for 30 minutes before addition of an EDC solution (10 µl, 1 mg). The mixture was incubated with slow tilt rotation at room temperature for 2 hours before blocking for 0.5 hour with ethanolamine in PBS (200 µl, 3 mg/ml). The conjugate was then washed and further blocked overnight with a solution of 1% BSA in PBS and stored in the same buffer at 4C.

### 4. Homogeneous magnetic beads based assay for C-Reactive Protein (CRP)

An Eppendorf tube (polypropylene, 500 µl) was sequentially loaded with the following reagents
A. 10 µl Magnetic Glass Bead-anti-CRP conjugate (10ug Beads)
B. 100 µl Buffer (PBS, 1%BSA)
C. 100 µl Anti-CRP-SERS conjugate in PBS, 1% BSA
D. 100 µl analyte solution (PBS, 1% BSA)

The mixture was incubated at room temperature by gentle mixing for 30 mn on mixing wheel. The magnetic particles were pulled to the bottom of the tube using a magnetic particle concentrator. The tubes were placed on the Raman reader and adjusted to position the bottom of the tube (i.e. magnetic beads) into the focal plane of the optical detection system. After a 1s reading time, the acquired data was reduced with a proprietary software package that allows analyte concentration determination.

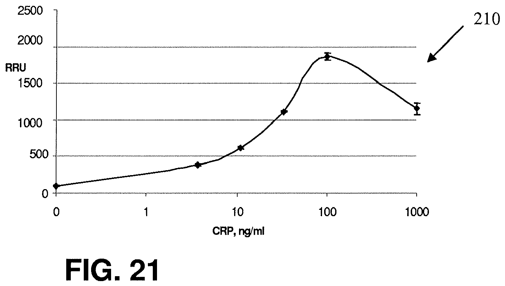

Data was collected using 1 um glass coated magnetic particles from Dynal, and bis(4-pyridyl)ethylene (BPE) SERS nanotags to detect CRP in buffer. The Raman spectra were collected on an Inspector Raman instrument from Delta Nu Corporation. The Inspector Raman system employs 40 mW, 785 nm laser excitation, free space optics, is portable and can be interfaced to a laptop computer. Data was analyzed using a software program performing least squares fitting of spectra to quantify components in complex mixtures. Graph 210 of Fig. 21 shows a dose response curve for CRP, a biologically relevant target for diagnoses of inflammatory and cardiovascular diseases. The assay is thus a one step, homogeneous, no wash procedure, performed in about 25 minutes. The observation that the SERS response at 1µg/ml CRP was lower than the response at 100ng/ml is attributed to the prozone or "hook effect", where excess analyte is saturating both capture and detection antibody sites thus prohibiting formation of the immune sandwich.

The dose-response in buffer described above was repeated several times with good assay reproducibility by giving standard deviation with coefficient of variation (cv) below 15% for triplicate reading of the same tube. The blank was read 20 times to give a cv of 20% and a intra-assay Limit of Detection of 200 pg/ml. This detection limit is more than 500 fold lower than the average normal physiological concentration of human CRP (100ng/ml).

SERS nanotags can be read in a background of biological matrix, such as plasma or blood. Fig. 9C shows the SERS spectra of a 70:30 mixture of whole blood and plasma in a glass vial. It is important to note that both neat plasma and whole blood have no significant Raman scattering intensity between 700 and 1800 wavenumbers. The top trace 90 of Fig. 9A shows the same spectra when SERS nanotags were added to the same biological cocktail. This illustrates the feasibility of reading SERS nanotags with adequate signal to noise ratios in biological matrices.

Not all conjugated antibodies remain active and not all particles remain colloidally stable after conjugation. Multiple attachment chemistries, multiple types of magnetic particles (differing by size, composition and surface functionality), multiple SERS tags functionalities and multiple antibodies for each analyte may be investigated.

It is important to develop robust and controllable methods of bioconjugation to both the capture and detection particle. Since suitable SERS nanotags and magnetic particles may be glass encapsulated it may be possible to use the same bioconjugation chemistries. The surface silanol groups can be easily derivatized with commercially available carboxy-, mercapto-, and aldehyde-silanes allowing a large optimization space to be explored. The preferred protocol for surface modification relies on thiolated SERS nanotags. Carboxylates, amines and aldehydes functionalities could give more stable intermediates. Different attachment chemistries (corresponding to different reaction conditions) may be investigated. This should cover buffer requirement (Pi) for most biological molecules and avoid potential for aggregation and denaturation during conjugation, as shown in Table 2 (Kricka (2005) Microchips, microarrays, biochips and nanochips - personal laboratories for the 21st century. http://www.ifcc.org/ejifcc/vol12no4/vol12no4a2.htm; MacBeath and Schreiber (2000) Science 289:1760-1763; Kusnezow and Hoheisel (2003) J. Molecular Recognition 16:165-176; Nemzek et al. (2001) J. Immunol. Methods 255:149-157). For example, reductive amination offers several advantages since it works over a wider pH range than the carbodiimide reaction (EDC), and therefore the pH can be controlled and adapted to the antibody stability/buffer requirement (Hermanson (1996) Bioconjugate Techniques. Academic Press). Antibody precipitation or particle aggregation could be avoided in this manner. Other advantages are that the amine bond is more stable than an amide bond, the overall charge of the antibody remains after reductive amination and there is no occurrence of antibody cross-linking as is very often observed during the carbodiimide reaction.

**Table 2: List of surface functionalization and conjugation chemistries.**

| *Particle functionality* | *Conjugation chemistry* | *Condition, pH* |
|---|---|---|
| Carboxylate | EDC | 5-7 |
| Thiol | Maleimide | 7-8 |
| Aldehyde | Reductive amination | 7-10 |

The major variable for is in the elimination of non-specific binding (NSB) and in an understanding of the interaction between the magnetic particle and SERS nanotag. The level to which each of the different conjugation protocols, and surface coverage, affect NSB may be investigated. The use of standard blocking agents and wash buffers (for example BSA, Tween, and casein, in addition to different pH and buffers) may be investigated. The time of reaction and concentration of particles will also be critical. It will be important that particles do not aggregate non-specifically. It is expected that non-specific binding will be minimized when the magnetic particle and SERS nanotag have the same surface characteristics. Should NSB prove insurmountable, polymer based derivatization of the SERS nanotag glass shell, such as polyacrylamide, polyethylene glycol and dextrans, polyethylenimine will be investigated (Lee et al. (2002) Bioconj. Chem. 13:97-103) and dendrimers (Le Berre et al. (2003) Nucleic Acids Res. 31:e88), all known to have low NSB. The optimized protocols of biofunctionalization of the SERS nanotags and magnetic particles by assessment of their pair-wise assay performance; determining sensitivity, dynamic range and specificity in buffer may be selected.

Glass-coated, superparamagnetic particles may be used as capture particles. Other particles commonly utilized for magnetic separation in molecular biology and readily available through companies such as Dynal, Bangs Laboratories, CPG Inc., and PerSeptive Biosystems may also be considered. These typical magnetizable particles have been extensively used in enzyme immunoassay (8) and for other binding assays in a wide size range (50nm to several micrometers). A 2.8 micron carboxylated polystyrene coated magnetic particle from Dynal has been compared with a 1 micron glass coated magnetic particle from Bioclone (Bioclone Magnetic Particles (2005) http://www.bioclon.com/functional-magnetic-paramagnetic-NH2-COOH-silica-epoxy-IDA-beads.html) and it has been found that the less porous glass-coated particles gave lower non-specific interaction of SERS nanotags in the CRP dose-response data. Particles can be obtained with different surface functional groups such as amine, carboxy, aldehyde, epoxy, silica, hydrazide, and others that have been used for stable and covalent attachment of antibodies.

Spectrally distinct SERS nanotags are manufactured in a highly reproducible manner (examples are illustrated in Fig. 2). Better detection limits may be achieved with future particles that are brighter and have uniform intensity.

The data discussed above was obtained in an eppendorf tube made of polypropylene. A major advantage of Raman analysis is that many materials of assay vessels used in the bioassay community are transparent to Raman light scatter, and the background Raman signal of these materials is in general minimal. The many proposed assay formats are not limited by the nature of the assay vessel (as is very often the case with fluorescence) but more importantly, the SERS signal can be read through the wall material of many assay vessels. The only limitation is that the container wall does not interfere/absorb the laser light energy (typically 650 or 783nm). A wide variety of inexpensive and commonly used assay vessels, including 96-well plates, tubes, vials, vacutainers and cups may be suitable for the described assays. The following material types: Glass, silica, silicon, silanized glass, polypropylene, polyethylene, polyurethane, Teflon, polystyrene, nitrocellulose, cellulose, polyester, and polycarbonate may be suitable for use in assay vessels.

### D. SERS / Magnetic Bead Hybridization and DNA Assay.

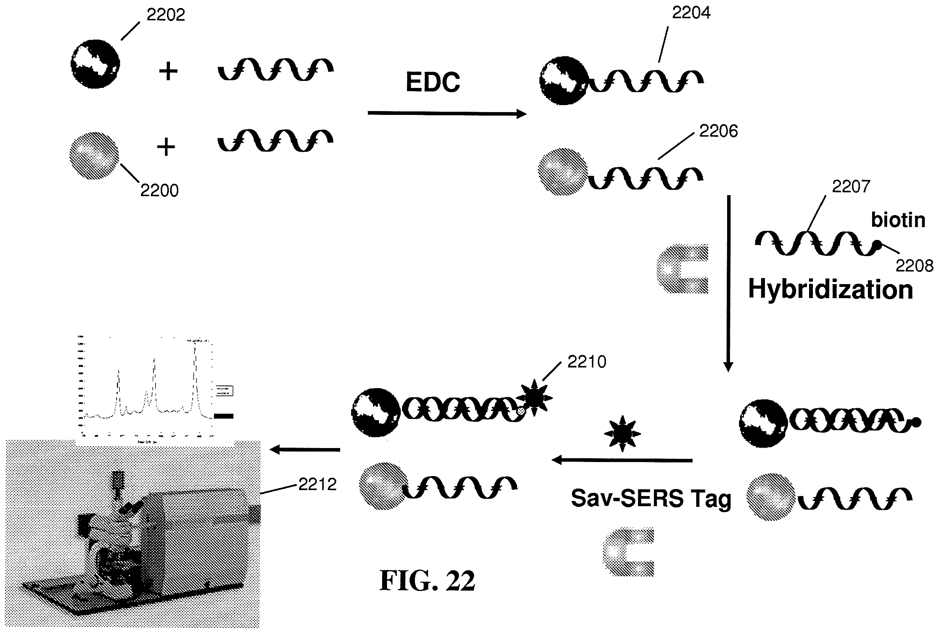

SERS nanotags and magnetic beads may be hybridized to function as a SERS magnetic DNA assay. Fig. 22 is a schematic diagram illustrating one method of SERS magnetic bead hybridization. Lighter spheres 2200 and darker spheres 2202 represent glass coated magnetic beads, with carboxyl surfaces. Two populations of beads are conjugated to two different oligonucleotide sequences 2204 and 2206 using standard EDC chemistry. Each population is separately hybridized with a complementary/non-complementary oligonucleotide sequence 2207 containing a biotin moiety 2208. Therefore, true multiplexing is not occurring at this point, rather each sequence is run in a separate eppendorf tube. The process described is applicable to bead hybridization for a multiplexed test as well. A SERS nanotag 2210 conjugated with streptavidin is added to the reaction, and a magnetic field used to isolate the pellet. In initial experiments the pellet was read using a Raman reader 2212 such as a Renishaw In Via Raman Microscope. Alternatively a less expensive or portable Raman inspection device may be utilized. A representative assay is described in Example 3 below. Other methods are equally suitable for preparing or performing an assay consistent with this aspect of the present disclosure.

An advantage of a biotin:streptavidin hybridization approach is that the target sequence being interrogated does not need to be labeled with SERS tag, but may be labeled with biotin instead. However, multiplexing is difficult. It may be desirable to employ a three-piece ligation to allow multiplexing. Alternatively, the conjugation order may be reversed with the original sequences first attached to the different "flavors" of SERS nanotags, followed by streptavidin coating of the magnetic particles.

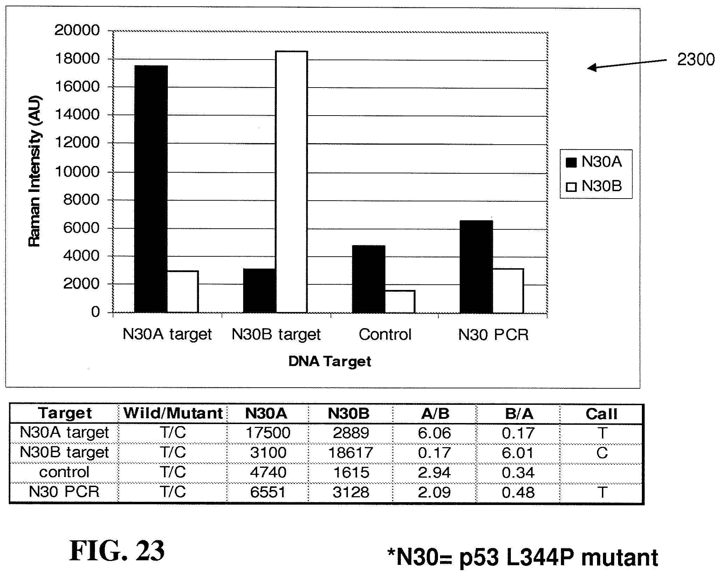

Graph 2300 of Fig. 23 illustrates the detection of p53 mutant DNA with a SERS magnetic bead system as described above. The assay of Figs. 22 and 23 is an unoptimized assay. Two variants of an oligonucleotide sequence selected from the L344P mutant in the p53 gene were designed, designated N30A and N30B. The target sequence for the N30A and N30B variants are as follows:

**Table 3**

| **Oligo Name** | **Design** | **Wild/Mutant** | **Codons Change** | **Sequence** | **Tm** |
|---|---|---|---|---|---|
| N30A | probe | p53 L344P | CTG-CCG Leu-Pro | amine-C12-TTTTTTTTTTTTTTTTTT CCGAGAGC**T**GAATGAG | 70 |
| N30A-T | target | | | 5' biotin CTCATTCAGCTCTCGG | 70 |
| N30B | probe | p53 L344P | CTG-CCG Leu-Pro | amine-C12-TTTTTTTTTTTTTTTTTT CCGAGAGC**C**GAATGAG | 72 |
| N30B-T | target | | | 5' biotin CTCATTCGGCTCTCGG | 72 |

In tube 1 N30A target was added to magnetic beads conjugated to N30A sequence (shown on the left of the N30A designated results of Fig. 23), and in tube 2 N30A target was added to magnetic beads conjugated to N30B sequence (shown to the right). Significant signal was observed only when N30A and N30A target were both present, with less signal from the non-complementary N30B sequence. In tube 3 N30B target was added to magnetic beads conjugated to N30A sequence (shown on the left of the N30B designated results of Fig. 23), and in tube 4 N30B target was added to magnetic beads conjugated to N30B sequence (shown on the right). Significant signal was observed only when N30B and N30B target were both present, with less signal from the non-complementary N30A sequence. A control experiment was performed (third two bars in graph 2300) in which no target was added, and as expected low signal was observed.

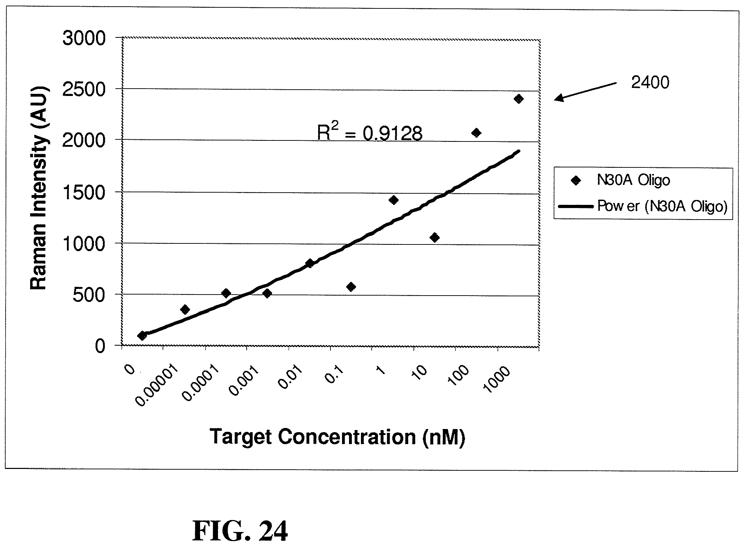

Graph 2400 of Fig. 24 graphically illustrates the results of a Titration of a SERS DNA Magnetic Assay as described above. A titration of N30A oligonucletoide target doped into N30A conjugated magnetic beads was performed, to determine potential limits of detection.

### Example 3:

### SERS Magnetic Bead Hybridization and DNA assay

### CONJUGATION

1. Take 40 ul magnetic beads (8X10 ⁷). Wash the beads with 50 mM MES (pH 4.5) twice
2. Re-suspend the beads with 163 ul 50 mM MES (pH4.5) and add 2 ul 50 uM oligo (100 pmole), put it on ice.
3. Make 20% EDC in 50 mM MES (pH7.0) (10 mg EDC at 50 ul MES).
4. Add 15 ul 20%EDC to the beads/oligo, Mix well and shaking for 1 hr at 4C
5. Wash with 500 ul 10mM PBS for four times. It is very important to wash out all un-conjugation oligo.
6. Resuspend in 40 ul PBS (beads 8X10 ⁷)

### HYBRIDIZATION

7. Take 33 ul 2xTMAC buffer, add 5 ul 10 uM oligo and 5 ul magnetic bead probe (4x10 ⁶) Hybridization at 55C for 1 hr
8. Wash with 1X SSC at room temperature for 5 min and 0.1X SSC at 55C for 5 min.
9. Add 1x NeutrAvidin -BPE (SERS tag) and incubated for 30 min.
10. Wash with 10 mM PBS twice
11. Acquire the Raman signal by Reinshaw InVia Raman microscope

### E. Competitive SERS / Magnetic Bead Assay.

A homogenous two particle competitive assay consistent with the present invention may be implemented with the use of SERS Nanotag detection particles and a separate capture particle. Certain advantages described below are realized if the capture particles are magnetic, but the scope of the invention is not limited to magnetic capture. A competitive assay format may be selected to implement any type of bioassay diagnostic test. The competitive format is particularly advantageous however, for the detection of analytes which are too small to accommodate two different antibodies capable of forming a sandwich type immune complex as described above. A competitive assay format as described herein may be utilized to detect any type of analyte including but not limited to therapeutic drugs, non-therapeutic drugs (drug abuse monitoring), peptides, hormones, proteins, or other analytes of interest. The competitive assay format is also particularly advantageous where only one binding agent is readily available for the analyte of interest.

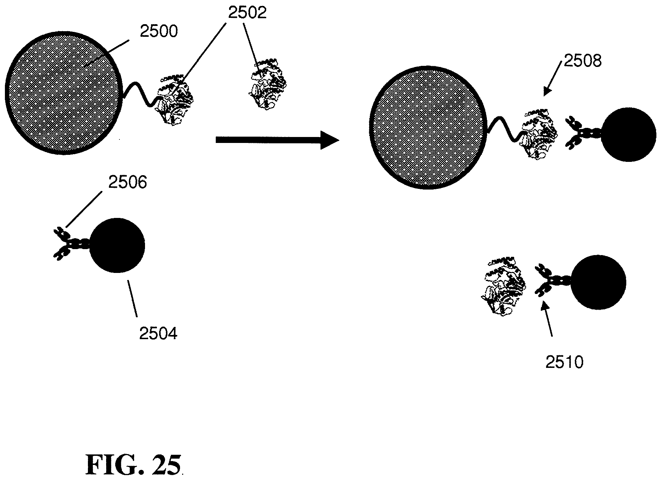

Certain specific competitive assay formats featuring capture particles and SERS nanotag detection particles are described in detail below. In general, however, all competitive assay formats require the labeling of either the capture particle or the detection particle with the analyte or an analog thereof. The detection particle may be optically labeled such as fluorescent, colored or SERS active particle. The particle not labeled with the analyte or an analog thereof is conjugated to a binding agent suitable for binding the analyte. For example, as is shown in Fig. 25A, a capture particle 2500 may be derivatized with an analyte of interest 2502. In addition, a SERS nanotag detection particle 2504 may be conjugated with a binding agent 2506 capable of selectively binding the analyte 2502. As is shown in Fig. 25A, contacting a sample containing free analyte 2502 with capture particles and SERS nanotag detection particles prepared as described above will result in the formation of a capture particle/SERS detection particle complex 2508 plus SERS detection particles bound to free analyte 2510.

The capture particles 2500 may be magnetic capture particles or other particles which are suitable for concentration. In one non-magnetic embodiment, the capture particles are large or dense particles pulled down by centrifugation. Large and/or dense capture particles include, but are not limited to, Nanobarcodes® Particles, spherical or anisotropic metallic nanoparticles, or any type of particle consisting of, but not limited to, latex, polystyrene, or other material, with sufficient specific gravity, size, and shape to be pulled down by centrifugation in the solvent of interest. In the Fig. 25A example, the capture particle/SERS detection particle complex 2508 may be concentrated (with a magnetic concentrator if the capture particle is a magnetic particle) and the Raman spectrum of a Raman reporter molecule associated with the SERS Nanotag detection particles may be acquired. The Raman signal intensity will be inversely proportional to the concentration of free analyte present in the sample since free analyte 2502 will competitively bind some of the conjugated SERS nanotag detection particles. As the concentration of free analyte increases, proportionately fewer SERS nanotag detection particles bind to the derivatized magnetic capture particles.

### Example 4:

### A. Competitive Assay Format with Simultaneous Reagent Addition

In a first competitive format, the capture and detection particles are added in rapid sequence to the analyte containing sample. During the incubation, both free analyte or an analog of the analyte either of which is referred to as "A" and particle-conjugated analyte may compete for conjugated binding agent "R". The binding agent "R" may have a different affinity for an analog A (preferably lower) compared to the affinity for endogenous A. After incubation, the magnetic particles (including free MP particles and MP-R::A-SERS or SERS-R::A-MP complexes depending upon which particle was conjugated with the binding agent) may be concentrated at a determined location within the reaction vessel followed by Raman spectrum acquisition. The Raman signal intensity is inversely proportional to the concentration (amount) of free (endogenous) analyte present in the sample. The possible complexes formed in this competitive assay, depending on which particle is derivatized with the analyte or analog are:

MP-A + A + SERS-R → SERS-R::A + SERS-R::A-MP; OR

SERS -A + MP-R + A → MP-R::A + MP-R + SERS -A + MP-R::A-SERS

### B. Pseudo-Competitive Assay Format with Sequential Reagent Addition

In a second competitive assay format, the analyte containing sample is first incubated with the particle conjugated to the binding agent "R" (MP-R or SERS-R) for a given time to allow for capture of endogeneous analyte "A" before addition of the particle conjugated to A. The later added analyte-derivatized particle then binds to the residual particle-R conjugate (MP-R or SERS-R). After incubation, the magnetic particles (free MP particles and MP-R::A-SERS or SERS-R::A-MP complexes) are concentrated at a determined location within the reaction vessel followed by Raman spectrum acquisition. The spectroscopically determined number of MP-R::A-SERS or SERS-R::A-MP is inversely proportional to the concentration of free (endogeneous A) present in the sample. The two step pseudo-competitive process includes the following reactions, depending upon which particle is conjugated with the binding agent.

SERS-R + A → SERS-R::A + SERS-R

(SERS-R::A + SERS-R) + MP-A → SERS-R::A + SERS-R::A-MP; OR

MP-R + A → MP-R::A + MP -R

(MP -R::A + MP-R) + SERS -A → MP-R::A + MP-R::A-SERS

In yet another assay format, SERS-R::A-MP (or MP-R::A-SERS) may be preformed and stored in the assay vessel in either liquid or lyophilized form. Upon the addition of sample, this particle-particle complex dissociates by interaction with endogeneous A in proportion to the analyte concentration, according to the following reactions.

MP-R::A-SERS + A → MP-R::A + MP-R::A-SERS; OR

SERS-R::A-MP + A → SERS-R::A + SERS-R::A-MP

### F. Nucleotide Sequence Detection Assays.

SERS Nanotag detection particles and separate capture particles may also be used to implement varied types of assays for the detection of nucleotide sequences. The nucleotide sequences which are suitable for detection by an assay as described herein may be from any source or organism. For example, nucleotide sources include but are not limited to human, animal, plant, bacteria, viral or other genetic material. Certain advantages as described below are realized if the capture particles are magnetic. The nucleotide sequences detection assays described herein allow for the detection of target nucleotide sequences in crude lysates and impure DNA preparations without the need for washing steps or the removal of sample mixtures. Certain assays described herein can be completed in a short time, for example, a half hour or less.

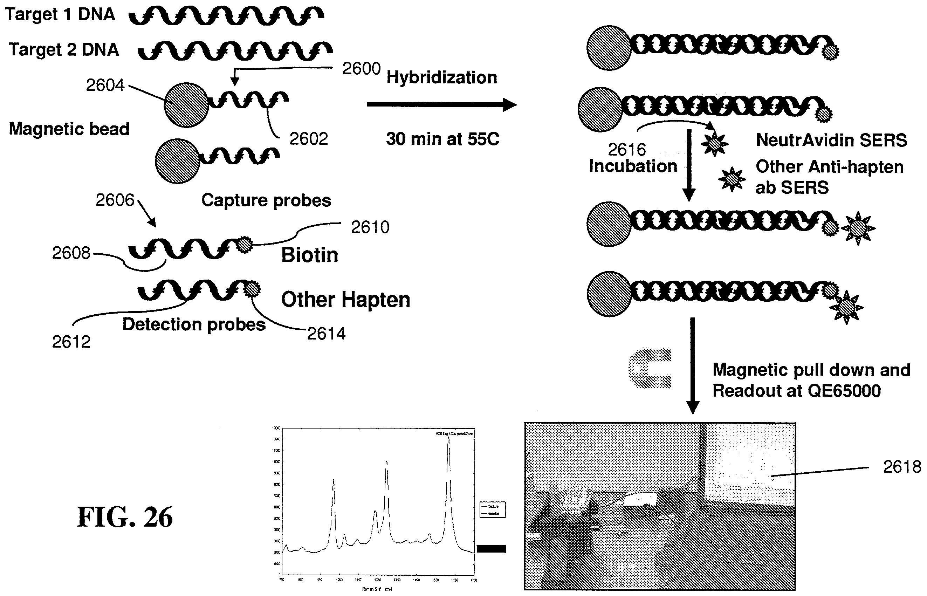

One nucleotide sequences detection assay consistent with the present disclosure is schematically illustrated in Fig. 26. The Fig. 26 assay features at least one capture probe 2600 which includes a capture nucleotide sequence 2602 conjugated to a particle. The selection steps described below can be optimized if the particle is a magnetic bead, paramagnetic bead or other magnetic particle 2604. This assay also includes a nucleotide sequences detection probe 2606 including a detection sequence 2608 conjugated to a capture molecule 2610. Both the capture sequence and the detection sequence are selected to have hybridization affinity for the nucleotide sequence of interest. The capture molecule may be any molecule suitable for binding a complementary target. For example, the detection probe 2606 may be a biotinylated or otherwise haptenylated sequence. A multiplexed assay may be prepared by providing separate detection probes 2606 with a first detection sequence 2608 being conjugated to a first capture molecule 2610 such as biotin and a second detection sequence 2612 being conjugated to a different capture molecule 2614 such as another hapten. It is important to note that this assay may be implemented with any capture molecule 2610 capable of binding a complementary target. The foregoing examples using biotin and another hapten are presented for illustrative purposes only.

The assay illustrated on Fig. 26 may be performed by hybridizing the capture probe and detection probe in the presence of the nucleotide sequences of interest. In addition, the hybridized complex may be incubated or otherwise contacted with a SERS nanotag 2616 conjugated to a complementary target molecule capable of selectively binding the capture molecule 2610. For example, the SERS nanotag may be conjugated to NeutrAvidin if biotin was selected as the capture molecule.

After incubation of the hybridized probes with the SERS nanotag complex, the Raman spectrum of a Raman reporter molecule associated with the SERS nanotag may be detected with a Raman spectrometer, Raman microscope or other detection device 2618. Concentration or selection of the resulting hybridized complex must be accomplished to separate hybridized nanotags from unbound nanotags. If a magnetic particle 2604 was used to prepare a capture probe, selection of the magnetic particles may be accomplished using known magnetic concentration techniques.

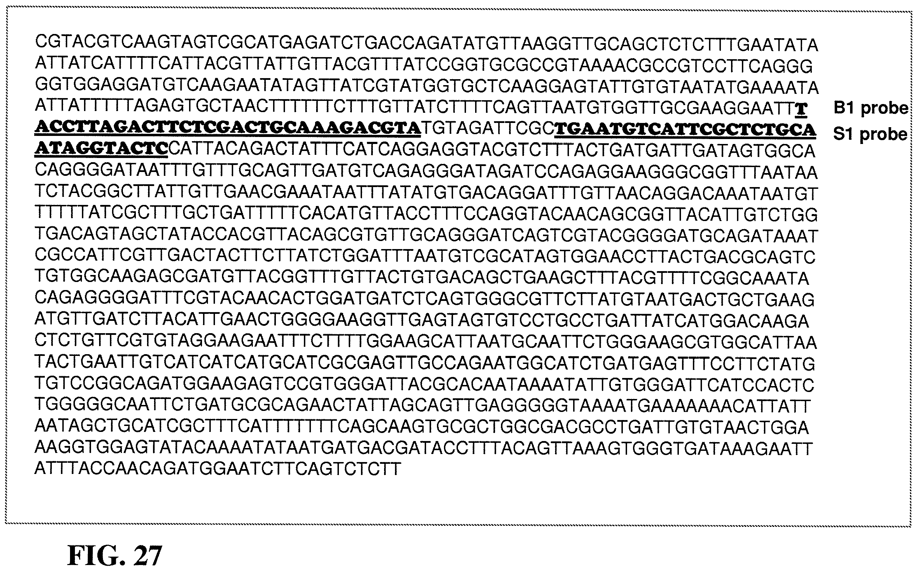

The data of experimental validation of the effectiveness of the Fig. 26 assay are shown graphically in Figs. 27-32. The experimentally determined limit of detection of the assay was around 4 pM.

Figure 27 is a detailed probe design suitable for use in a nucleotide sequenced detection assay. The B1 probe may be conjugated to magnetic particle 2604 and the S1 probe may be conjugated to SERS tag 2616. A hybridization would be processed when a B probe, an S probe and a target are present in suitable conditions, for example 55C.

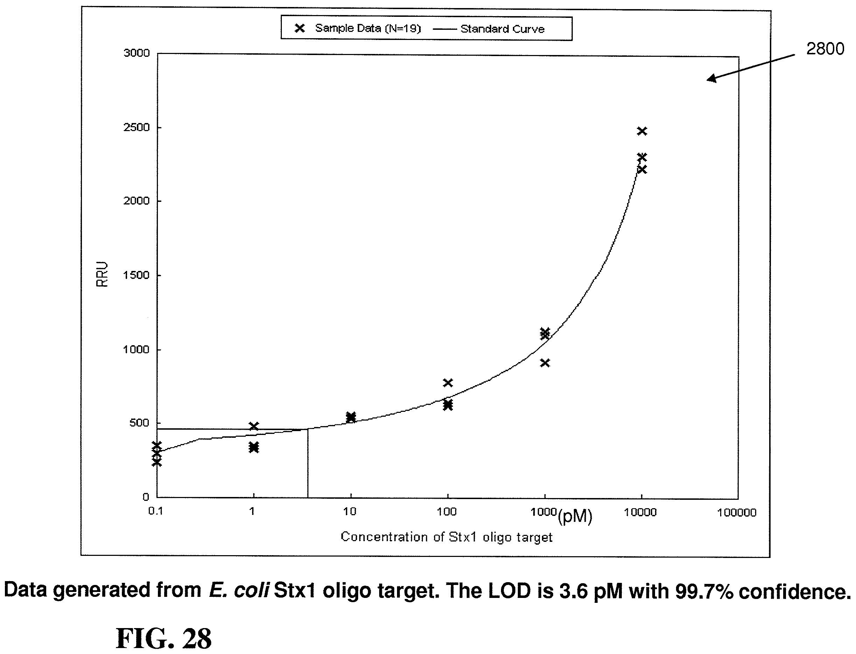

Figure 28 illustrates a graph 2800 showing proof of concept of the assay shown in Fig. 26. *E. coli* target gene Stex1 oligo was diluted from 1000 pM to 1 pM. The data indicates that limit of detection is about 4 pM with 99.7% confidence.

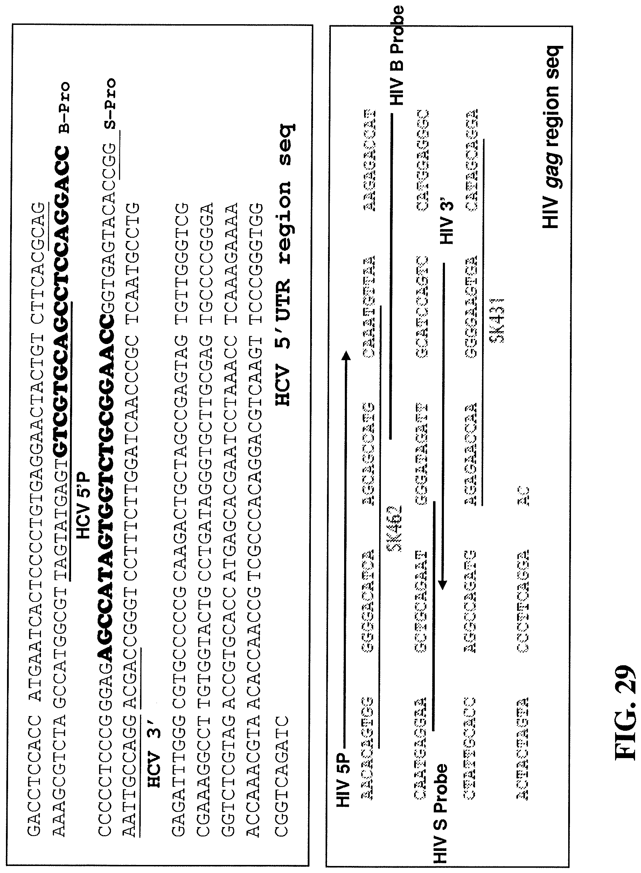

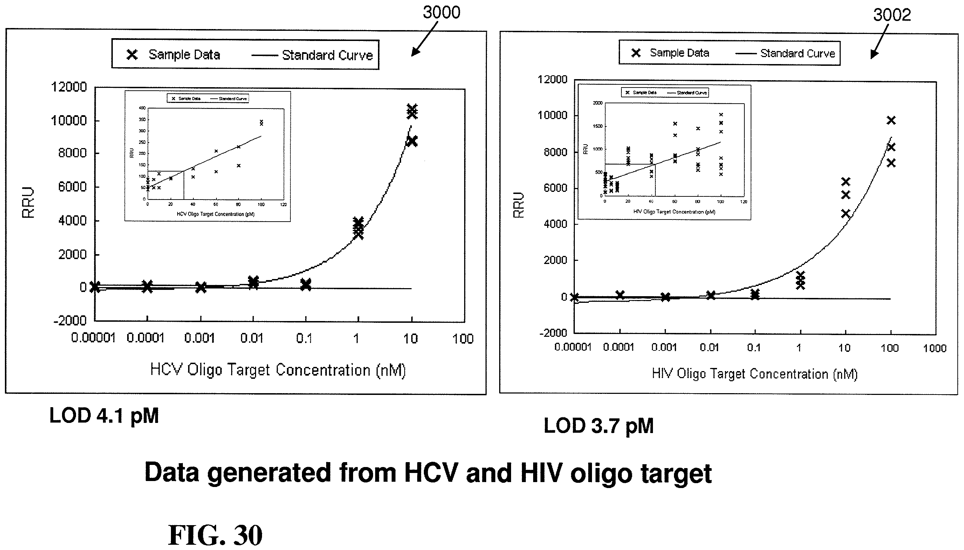

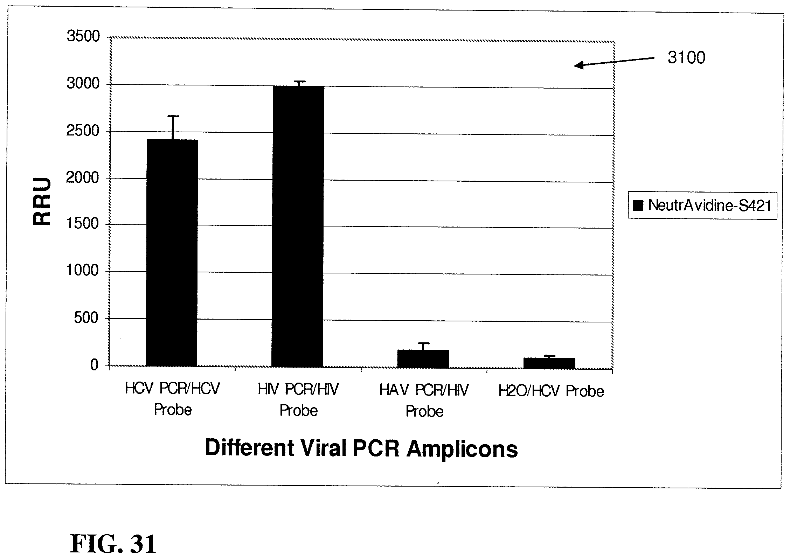

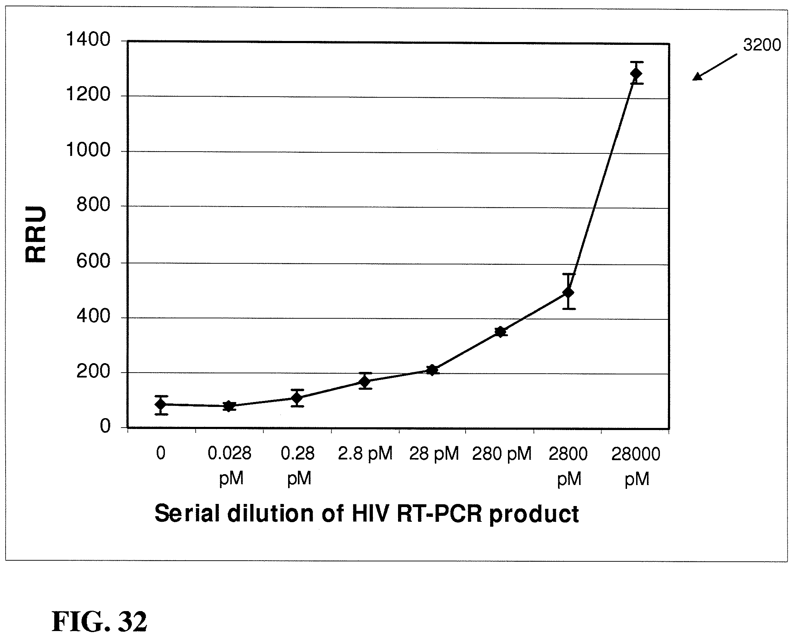

Figure 29 illustrates certain RT-PCR primers and hybridization probes designed for HIV and HCV detection. For example, the HCV 5'p and HCV 3' probe may be used for RT-PCR. The illustrated B probe may be conjugated to a magnetic particle such as magnetic bead 2604 and the illustrated S probe conjugated to a SERS tag 2616. Graphs 3000 and 3002 of Figure 30 illustrate the results of a titration for HCV (3000) and HIV (3002). Also illustrated in Fig. 30 are a linear correlation between the target concentration and signal. The limit of detection for HCV was shown to be about 4.1 pM and HIV was shown to be about 3.7 pM. In order to confirm the effectiveness of this assay on a clinical sample, HCV or HIV RNA samples were amplified by RT-PCR and these products were detected by this system. The results illustrate that an HCV probe as described herein was suitable to detect the HCV sequence-specific targets. False detection of a wrong target such as HAV or a negative control was not observed. (See Graph 3100 of Figure 31.) Similar results were obtained when detecting HIV. See for example Graph 3200 of Fig. 32. The result indicated that the limit of detection for RT-PCR product is about 0.28 -2.8 pM. This confirms that the described assay can detect a DNA target concentration as low as 0.28 - 4 pM.

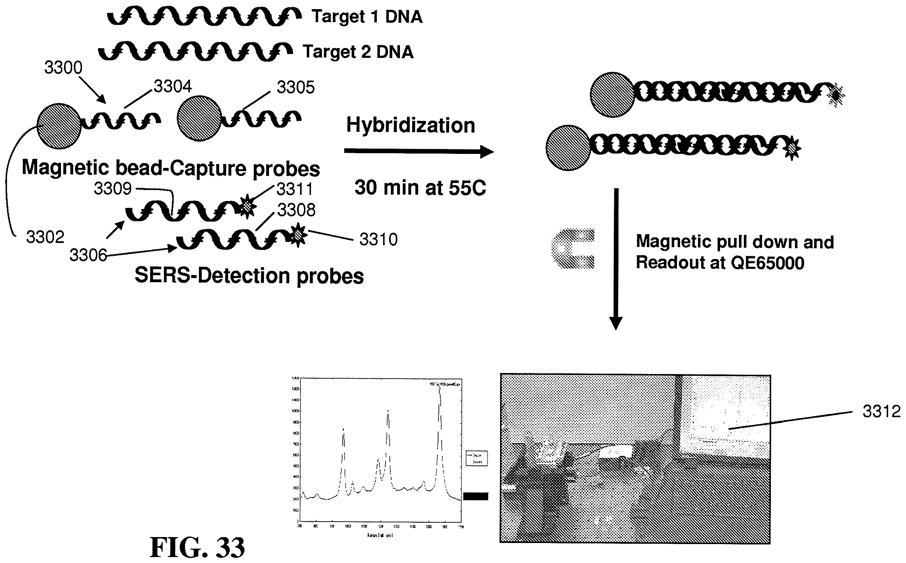

An alternative nucleotide sequences detection assay is schematically illustrated in Fig. 33. In the Fig. 33 assay, one or more types of capture probe 3300 are provided by conjugating a particle which may be a magnetic particle 3302 to a capture sequence 3304 or 3305. In addition, one or more types of detection probes 3306 may be provided by directly conjugating a detection sequence 3308, 3309 to a SERS nanotag 3310 or 3311. In each case, the capture and detection sequences are selected to have hybridization affinity for the target nucleotide sequences. The capture probes 3300 and detection probes 3306 may be hybridized in the presence of the nucleotide sequences of interest. Subsequently, the resulting complex may be selected or concentrated followed by the detection of the Raman spectrum of a Raman reporter molecule associated with the SERS nanotag. If a magnetic particle 3302 is used to prepare the capture probes 3300, the complex of interest may be selected or concentrated using known magnetic concentration techniques. A multiplexed test may be prepared by providing multiple capture probes 3300 featuring distinct capture sequences 3304, 3305 and multiple detection probes 3306 with distinct detection sequences 3308, 3309 with each type of detection sequence being conjugated to a type of SERS nanotags 3310, 3311 featuring a distinct Raman reporter molecule.

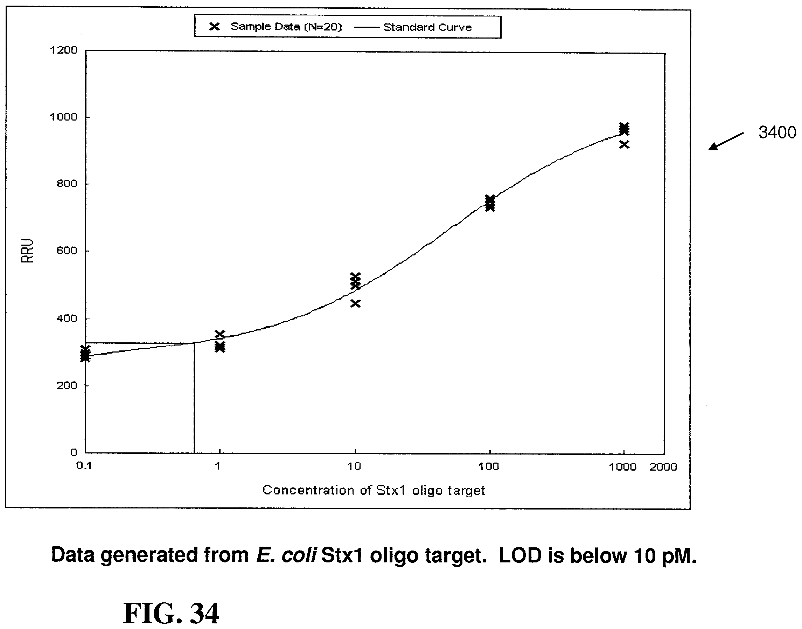

Graph 3400 of Fig. 34 is a representation of the results of experimental validation of the assay illustrated in Fig. 33. The limit of detection in a mimic oligonucleotide system was around 10 pM under unoptimized assay conditions. The limit of detection in model systems is decreased when hybridization occurs in the presence of unrelated genomic DNA as background. Detectability may be further reduced when the oligonucleotide probe hybridizes to a mega-base genomic DNA target instead of the desired mimic oligo target. Accordingly, it may in certain instances be desirable to increase the efficiency and sensitivity of an assay.

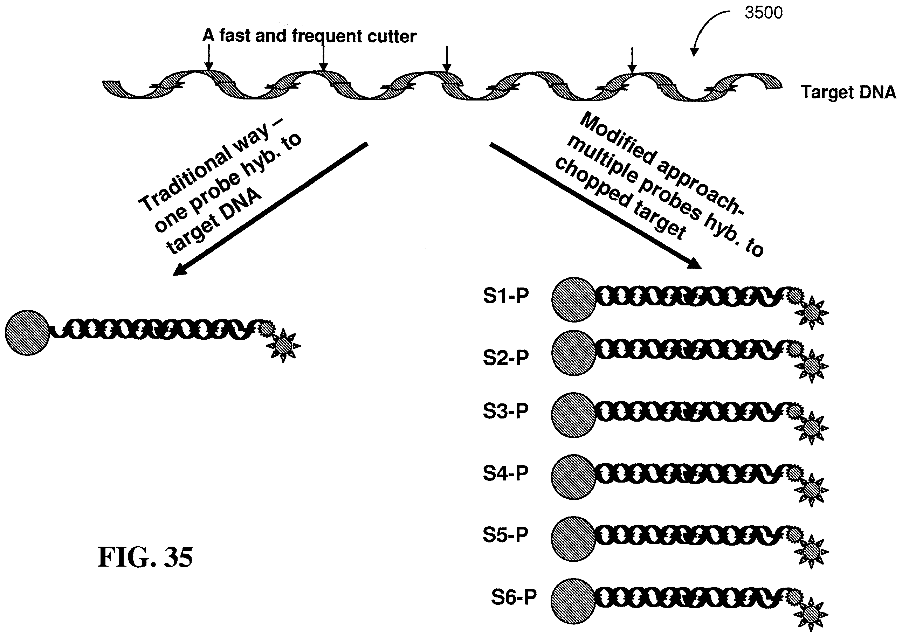

One method of increasing nucleotide assay sensitivity is schematically illustrated in Fig. 35. This assay includes an initial cutting or breaking of the nucleotide sequence of interest 3500 into multiple sections. Any known technique to cut a nucleotide into sections may be employed in this step. For example, a frequent and fast-acting restriction enzyme may be used to digest the nucleotide of interest into small pieces. The thus prepared sections of the target nucleotide may be then hybridized with multiple distinct capture probes and multiple distinct detection probes, with each type of probe having distinct hybridization affinities for separate portions of the nucleotide sequences of interest. As is described above, the detection probes may be conjugated to a SERS nanotag and the capture probes conjugated to a magnetic particle. The fragmentation of the target DNA into multiple small pieces effectively increases hybridization efficiency and target complex concentration, since the multiple fragments of the desired target present in the assay sample may be separately bound to multiple detection particles which increases the signal and therefore increases assay sensitivity. An advantage associated with the method illustrated in Fig. 35 is that this method increases assay sensitivity without the introduction of amplification bias.

Alternatively, a larger nucleotide fragment (for example, a genomic or gene fragment without digestion or with minimal digestion) can be hybridized with a capture sequence conjugated to a capture particle. Furthermore, multiple different detection sequences complementary to the large nucleotide fragment may each be conjugated with same SERS nanotag. Assay sensitivity will thus increase by increasing the number of detection probes on a single larger nucleotide sequence. In this case the assay specificity could also be increased by using a plurality of SERS nanotags. Alternatively, if multiple different detection sequences have been conjugated with different SERS nanotags, or a specific genomic deletion or mutation can be identified by designing specific detection sequences to detect the region of interest. Furthermore, the magnetic particle could be conjugated to multiple different capture sequences complementary to different nucleotide fragments of interest resulting in the assay signal being enhanced for each magnetic particle.

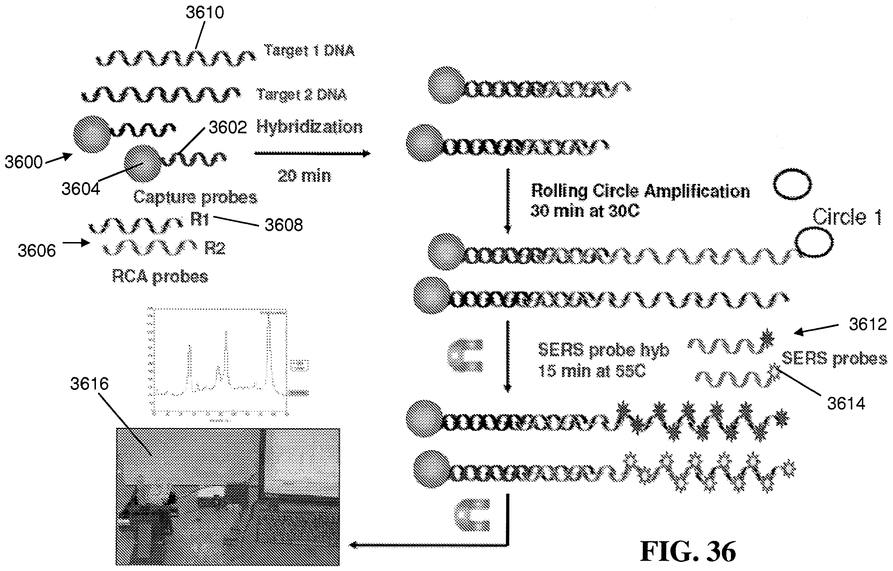

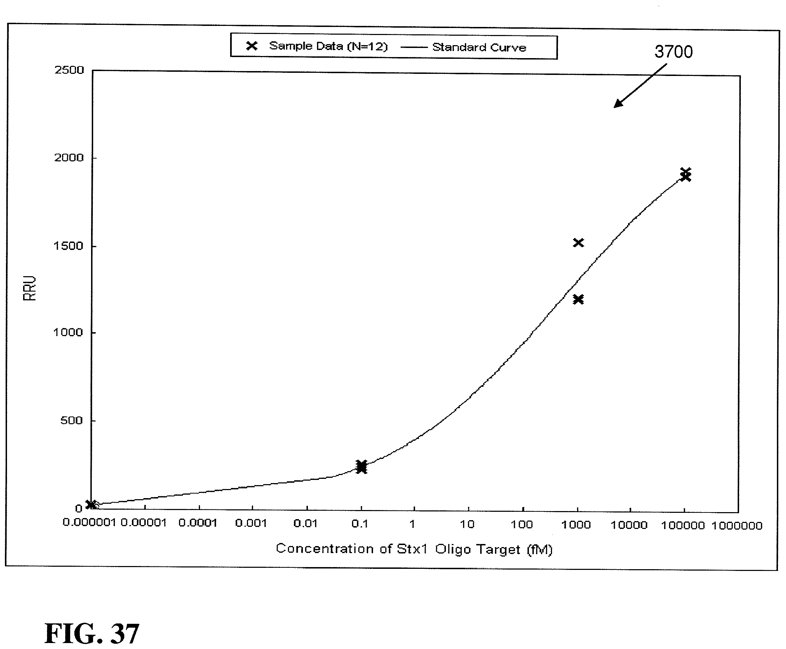

Other methods may be used to increase nucleic acid assay sensitivity. For example, isothermal rolling circle amplification (RCA), polymerase chain reation (PCR) related amplification, T7 RNA polymerase-driven amplification , ligase chain reaction (LCR), branched DNA (bDNA) or other known amplification methods may be used in conjunction with capture and detection probes as described above. An example assay featuring RCA is illustrated in Fig. 36. The Fig. 36 assay has capture probes 3600 featuring a capture sequence 3602 conjugated to a magnetic particle 3604 and one or more RCA probes 3606 each conjugated to an RCA molecule 3608 which may be hybridized in the presence of a nucleotide sequence of interest 3610 followed by RCA using known techniques. The resulting complex may be contacted with one or more detection probes 3612 each conjugated with a SERS nanotag 3614 thus substantially increasing the number of nanotags associated with each capture molecule. Subsequently, the Raman spectrum of a Raman reporter molecule associated with the SERS nanotag may be acquired using a Raman spectrometer, Raman microscope or other apparatus 3616. Graph 3700 of Fig. 37 illustrates the sensitivity of an assay prepared in accordance with Fig. 36. The Fig. 37 assay format is described in detail in Example 5 below. Those skilled in the art will note that in an RCA enhanced assay, the magnetic capture particle associated with the capture probe may be used to facilitate selection and/or concentration both after RCA and after incubation of the initial hybridization products in the presence of SERS nanotag detection probes.

### Example 5:

### SERS Tag Glass-coating Carboxyl Functionalization

### AMINE FUNCTIONALIZATION:

1. Wash 500 µl 20x glass-coated SERS tags three times in EtOH.
2. Re-suspend in 930 µl EtOH. Add 50 µl water and 20 µl APTMS.
3. Mix for one hour.
4. Wash three times in 1 ml DMSO.

### Carboxyl functionalization:

5. Dissolve 0.04 g succinic anhydride in 1 ml DMSO.
6. Add 10 µl succinic anhydride solution to SERS tags in DMSO. Mix for one hour.
7. Add 10 µl more succinic anhydride solution. Mix for one more hour.
8. Rinse three times in 50 mM MES pH4.5.
9. Re-suspend the SERS tags at 500 µl MES and store at 4C.

### CONJUGATION OF OLIGO TO FUNCTIONALIZED SERS TAGS

1. Take 40 µl 20x functionalized Sers tag. Wash it with 50 mM MES (pH 4.5) twice.
2. Re-suspend the Sers tag with 163 µl 50 mM MES (pH4.5) and add 2 µl 50 µM oligo (100 pmole), put it on ice.
3. Make 20% EDC in 50 mM MES (pH7.0) (10 mg EDC at 50 µl MES).
4. Add 15 µl 20%EDC to the Sers tag/oligo. Mix well and shake for 1 hour at 4C.
5. Wash with 500 µl 10mM PBS four times. It is very important to wash out all un-conjugation oligo.
6. Re-suspend in 40 µl PBS.

### CONJUGATION OF OLIGO TO MAGNETIC BEADS

1. Take 40 µl magnetic beads (8X10 7). Wash the beads with 50 mM MES (pH 4.5) twice.
2. Re-suspend the beads with 163 µl 50 mM MES (pH4.5) and add 2 µl 50 µM oligo (100 pmole), put it on ice.
3. Make 20% EDC in 50 mM MES (pH7.0) (10 mg EDC at 50 µl MES).
4. Add 15 µl 20% EDC to the beads/oligo. Mix well and shake for 1 hour at 4C.
5. Wash with 500 µl 10mM PBS four times. It is very important to wash out all un-conjugation oligo.
6. Re-suspend in 40 µl PBS (beads 8X10 7).

### HYBRIDIZATION

1. Take 33 µl 2xTMAC or HS 114 buffer, add 5 µl 10 µM oligo and 5 µl magnetic bead probe (4x10 6) Hybridization at 55C for 30 minutes. Either acquire the Raman signal or continue the following steps.
2. Wash with 1X SSC at room temperature for 5 minutes and 0.1X SSC at 55C for 5 minutes.
3. Add 1x NeutrAvidin -BPE (SERS tag) and incubate for 30 minutes.
4. Wash with 10 mM PBS twice.
5. Acquire the Raman signal with Reinshaw InVia Raman microscope or QE65000 spectrometer.

### SERS ROLLING CICLE AMPLIFICATION:

1. In 42 µl HS 114 buffer, add 5 µl oligo, 1 µl 5uM RCA probe and 2 µl beads-probe to a total volume of 50 µl and incubate at 55C for 30 minutes.
2. Wash with PBS once, and add 46 µl PBS/0.05% Tween-20 and 4 µl 10 µM circle1 probe to the beads.
3. Incubate at 37C for 30 minutes and wash with PBS once.
4. To the beads, add 2 µl phi129 DNA polymerase (20U), 1 µl dNTP (20 mM/each dNTP), 10 µl 2x buffer/1x BSA and 7 µl H2O, total up to 20 µl.
5. Incubate at 37 C for 2 hours to overnight. Wash with PBS once.
6. Add 50 µl 0.5X SERS tag-probe in 2x SSC/0.05% Tween-20 solution to the beads and incubate at 37C for 20-30 minutes.
7. Wash with 1X SSC for 1 minute at room temperature.
8. Wash with 0.1X SSC for 1 minute at room temperature.
9. Acquire the Raman signal on QE65000.

## Claims

1. A method of detecting an analyte of interest comprising:
providing capture particles derivatized with said analyte;
providing SERS nanotag detection particles conjugated with a first molecule capable of selectively binding said analyte;
contacting a sample which may contain the analyte of interest with the capture and SERS nanotag detection particles, thereby forming both an analyte/SERS nanotag detection particle complex and a capture particle/SERS nanotag detection particle complex;
concentrating the capture particle/SERS nanotag detection particle complex; and
detecting the Raman spectrum of a Raman reporter molecule associated with the SERS nanotag detection particles.

2. The method of claim 1, wherein the capture particles are magnetic particles.

3. The method of claim 2, wherein the concentration step is performed on a magnetic particle concentrator.

4. An assay system for detecting an analyte of interest according to a method as defined in claim 1, comprising:
capture particles derivatized with said analyte;
SERS nanotag detection particles conjugated with a first molecule capable of selectively binding said analyte;
optionally wherein the capture particles are magnetic particles.

## Patentansprüche

1. Verfahren zum Erfassen eines Analyten von Interesse, umfassend:
Bereitstellen von mit dem Analyten derivatisierten Fängerpartikeln;
Bereitstellen von mit einem ersten Molekül, das in der Lage ist, den Analyten selektiv zu binden, konjugierten SERS-Nanokennzeichnung-Erkennungspartikeln;
Inkontaktbringen einer Probe, welches den Analyten von Interesse enthalten kann, mit den Fänger- und SERS-Nanokennzeichnung-Erkennungspartikeln, dadurch Bilden von sowohl einem Analyt/SERS-Nanokennzeichnung-Erkennungspartikel-Komplex als auch einem Fängerpartikel/SERS-Nanokennzeichnung-Erkennungspartikel-Komplex;
Konzentrieren des Fängerpartikel/SERS-Nanokennzeichnung-Erkennungspartikel-Komplexes; und
Erfassen des Ramanspektrums eines mit den SERS-Nanokennzeichnung-Erkennungspartikeln assoziierten Raman-Reportermoleküls.

2. Verfahren nach Anspruch 1, wobei die Fängerpartikel magnetische Partikel sind.

3. Verfahren nach Anspruch 2, wobei der Konzentrationsschritt in einem Konzentrator für magnetische Partikel durchgeführt wird.

4. Assaysystem zum Erfassen eines Analyten von Interesse nach einem Verfahren wie in Anspruch 1 definiert, umfassend:
mit dem Analyten derivatisierte Fängerpartikel;
SERS-Nanokennzeichnung-Erkennungspartikel, konjugiert mit einem ersten Molekül, das in der Lage ist, den Analyten selektiv zu binden;
wahlweise wobei die Fängerpartikel magnetische Partikel sind.

## Revendications

1. Procédé de détection d'une substance à analyser d'intérêt comprenant :
la fourniture de particules de capture dérivées avec ladite substance à analyser ;
la fourniture de particules de détection de nanomarqueur SERS conjuguées avec une première molécule capable de lier de façon sélective ladite substance à analyser ;
la mise en contact d'un échantillon qui peut contenir la substance à analyser d'intérêt avec les particules de capture et de détection de nanomarqueur SERS, en formant de ce fait à la fois un complexe substance à analyser/particule de détection de nanomarqueur SERS et un complexe particule de capture/particule de détection de nanomarqueur SERS ;
la concentration du complexe particule de capture/particule de détection de nanomarqueur SERS ; et
la détection du spectre de Raman d'une molécule rapporteuse de Raman associée aux particules de détection de nanomarqueur SERS.

2. Procédé selon la revendication 1, dans lequel les particules de capture sont des particules magnétiques.

3. Procédé selon la revendication 2, dans lequel l'étape de concentration est effectuée sur un concentrateur de particules magnétiques.

4. Système de dosage pour détecter une substance à analyser d'intérêt conformément à un procédé selon la revendication 1, comprenant:
des particules de capture dérivées avec ladite substance à analyser ;
des particules de détection de nanomarqueur SERS conjuguées avec une première molécule capable de lier de façon sélective ladite substance à analyser ;
éventuellement dans lequel les particules de capture sont des particules magnétiques.


<110> APPLICANT: Becton, Dickinson & Company
<120> TITLE OF INVENTION: SERS NANOTAG ASSAYS
<130> FILE REFERENCE: OXO13099PCTEPD1
<140> CURRENT APPLICATION NUMBER:
   <141> CURRENT FILING DATE: 2007-02-08
<150> PRIOR APPLICATION NUMBER: EP 07 763 259.4
   <151> PRIOR FILING DATE: 2007-02-08
<160> NUMBER OF SEQ ID NOS: 7
<170> SOFTWARE: PatentIn version 3.3
<210> SEQ ID NO 1
   <211> LENGTH: 34
   <212> TYPE: DNA
   <213> ORGANISM: Artificial
<220> FEATURE:
   <223> OTHER INFORMATION: Probe
<400> SEQUENCE: 1 
tttttttttt ttttttttcc gagagctgaa tgag
34
<210> SEQ ID NO 2
   <211> LENGTH: 16
   <212> TYPE: DNA
   <213> ORGANISM: Artificial
<220> FEATURE:
   <223> OTHER INFORMATION: Target
<400> SEQUENCE: 2 
ctcattcagc tctcgg
16
<210> SEQ ID NO 3
   <211> LENGTH: 34
   <212> TYPE: DNA
   <213> ORGANISM: Artificial
<220> FEATURE:
   <223> OTHER INFORMATION: Probe
<400> SEQUENCE: 3 
tttttttttt ttttttttcc gagagccgaa tgag
34
<210> SEQ ID NO 4
   <211> LENGTH: 16
   <212> TYPE: DNA
   <213> ORGANISM: Artificial
<220> FEATURE:
   <223> OTHER INFORMATION: Target
<400> SEQUENCE: 4 
ctcattcggc tctcgg
16
<210> SEQ ID NO 5
   <211> LENGTH: 410
   <212> TYPE: DNA
   <213> ORGANISM: Hepatitis C virus
<400> SEQUENCE: 5 
gacctccacc atgaatcact cccctgtgag gaactactgt cttcacgcag aaagcgtcta
60
gccatggcgt tagtatgagt gtcgtgcagc ctccaggacc ccccctcccg ggagagccat
120
agtggtctgc ggaaccggtg agtacaccgg aattgccagg acgaccgggt cctttcttgg
180
atcaacccgc tcaatgcctg gagatttggg cgtgcccccg caagactgct agccgagtag
240
 
tgttgggtcg cgaaaggcct tgtggtactg cctgataggg tgcttgcgag tgccccggga
300
ggtctcgtag accgtgcacc atgagcacga atcctaaacc tcaaagaaaa accaaacgta
360
acaccaaccg tcgcccacag gacgtcaagt tcccgggtgg cggtcagatc
410
<210> SEQ ID NO 6
   <211> LENGTH: 172
   <212> TYPE: DNA
   <213> ORGANISM: Human immunodeficiency virus
<400> SEQUENCE: 6 
aacacagtgg ggggacatca agcagccatg caaatgttaa aagagaccat caatgaggaa
60
gctgcagaat gggatagatt gcatccagtc catggagggc ctattgcacc aggccagatg
120
agagaaccaa ggggaagtga catagcagga actactagta cccttcagga ac
172
<210> SEQ ID NO 7
   <211> LENGTH: 1320
   <212> TYPE: DNA
   <213> ORGANISM: Artificial
<220> FEATURE:
   <223> OTHER INFORMATION: Probe
<400> SEQUENCE: 7

cgtacgtcaa gtagtcgcat gagatctgac cagatatgtt aaggttgcag ctctctttga
60
atataattat cattttcatt acgttattgt tacgtttatc cggtgcgccg taaaacgccg
120
tccttcaggg ggtggaggat gtcaagaata tagttatcgt atggtgctca aggagtattg
180
tgtaatatga aaataattat ttttagagtg ctaacttttt tctttgttat cttttcagtt
240
aatgtggttg cgaaggaatt taccttagac ttctcgactg caaagacgta tgtagattcg
300
ctgaatgtca ttcgctctgc aataggtact ccattacaga ctatttcatc aggaggtacg
360
tctttactga tgattgatag tggcacaggg gataatttgt ttgcagttga tgtcagaggg
420
atagatccag aggaagggcg gtttaataat ctacggctta ttgttgaacg aaataattta
480
tatgtgacag gatttgttaa caggacaaat aatgtttttt atcgctttgc tgatttttca
540
catgttacct ttccaggtac aacagcggtt acattgtctg gtgacagtag ctataccacg
600
ttacagcgtg ttgcagggat cagtcgtacg gggatgcaga taaatcgcca ttcgttgact
660
acttcttatc tggatttaat gtcgcatagt ggaaccttac tgacgcagtc tgtggcaaga
720
gcgatgttac ggtttgttac tgtgacagct gaagctttac gttttcggca aatacagagg
780
ggatttcgta caacactgga tgatctcagt gggcgttctt atgtaatgac tgctgaagat
840
gttgatctta cattgaactg gggaaggttg agtagtgtcc tgcctgatta tcatggacaa
900
gactctgttc gtgtaggaag aatttctttt ggaagcatta atgcaattct gggaagcgtg
960
gcattaatac tgaattgtca tcatcatgca tcgcgagttg ccagaatggc atctgatgag
1020
tttccttcta tgtgtccggc agatggaaga gtccgtggga ttacgcacaa taaaatattg
1080
tgggattcat ccactctggg ggcaattctg atgcgcagaa ctattagcag ttgagggggt

1140
    aaaatgaaaa aaacattatt aatagctgca tcgctttcat ttttttcagc aagtgcgctg
1200
    gcgacgcctg attgtgtaac tggaaaggtg gagtatacaa aatataatga tgacgatacc
1260
    tttacagtta aagtgggtga taaagaatta tttaccaaca gatggaatct tcagtctctt
1320